# EUROPEAN PATENT APPLICATION

(11) **EP 4 014 964 A1**
(43) Date of publication of application: **22.06.2022**
(21) Application number: 20383127.6
(22) Date of filing: 21.12.2020
(51) Int. Cl.: A61K 9/00, A61K 47/36, A61K 47/34, A61K 47/26, A61K 31/40

(54) **TOPICAL FORMULATION**

(71) Applicant: Almirall S.A., 08022 Barcelona (ES)
(72) Inventor: GARCIA GONZALEZ, Nuria, 08980 Sant Feliu de Llobregat BARCELONA (ES); GUIRO COLL, Pere, 08980 Sant Feliu de Llobregat BARCELONA (ES); EKELUND, Katarina, 08980 Sant Feliu de Llobregat BARCELONA (ES)
(74) Representative: Herrero & Asociados, S.L.

(57) **Abstract**

The present invention relates to a topical gel composition comprising:
(a) a pyrrole compound, which pyrrole compound is a compound of Formula (I) or a pharmaceutically acceptable salt thereof: 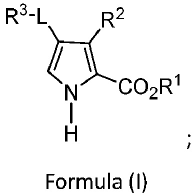 (b) water, and (c) a gelling agent. The compound of formula (I) has ACC inhibitory activity.

## Description

### Field of the invention

The present invention relates to a topical gel composition comprising a compound having ACC inhibitory activity.

### Background to the invention

Acetyl-CoA carboxylase (ACC) is the rate-limiting enzyme in de novo synthesis of fatty acids (Strable MS and Ntambi JM. Crit Rev Biochem Mol Biol. 2010;45:199-214) and in the translocation of fatty acids to the mitochondria for β-oxidation (Schreurs M et al. Obes Rev. 2010;11:380-8). ACC is also key for the elongation of fatty acids including essential fatty acids (Kim CW et al. Cell Metab. 2017;26:394-406). ACC catalyzes the ATP-dependent carboxylation of acetyl-CoA to malonyl-CoA (Barber MC et al. Biochim Biophys Acta. 2005 Mar; 1733: 1-28). In mammals, ACC activity is produced by two isoenzymes, namely ACC1 (also known as ACCα) and ACC2 (also known as ACCβ) encoded by two different genes (Acc1 and Acc2 respectively) (Barber MC et al. Biochim Biophys Acta. 2005 Mar; 1733: 1-28). ACC1 is located in the cytosol and is involved in the synthesis and elongation of fatty acids. ACC2 is located in cytosolic face of the external mitochondrial membrane and is involved in the inhibition of the carnitine palmitolyltransferase I (CPT-I), which is the crucial enzyme for the transport of long-chain fatty acids to mitochondria for β-oxidation (Tong L. Cell Mol Life Sci. 2013; 70: 863-91). The activity of both ACC1 and ACC2 in mammals is stimulated by citrate, inhibited by long chain saturated acyl-CoA and inactivated by phosphorylation, especially by AMP-activated protein kinase (AMPK) and cAMP-dependent protein kinase (PKA) (Brownsey RW et al. Biochem Soc Trans. 2006; 34: 223-7). ACC activity is also key for the survival of several organisms, some of them related to human pathologies such as bacteria, virus and parasites (Tong L. Cell Mol Life Sci. 2013; 70: 863-91). In several immune cells types, including T cells and macrophages ACC activity is required for the differentiation, survival and production of cytokines such as IL-17 (Buck M. et al. Cell. 2017; 169: 570-86). The crucial role of ACC enzymes in several (patho)physiological processes make them attractive pharmaceutical targets for diseases related to fatty acid metabolism alterations, dermatological diseases such as acne or psoriasis, diabetes, obesity, nonalcoholic steatohepatitis (NASH), cancer, atherosclerosis, inflammation, autoimmunity, infection, and infestation among others (Luo D. et al. Recent Pat Anticancer Drug Discov 2012; 7: 168-84). Indeed, several dermatological diseases are linked to ACC activity, for instance acne is characterized for an increase in sebum production (Pappas A. et al. Dermatoendocrinol. 2009; 1: 157-61; Williams H et. al. Lancet. 2012; 379: 361-72) and both T cells and IL-17 are increased in acne and psoriatic lesions (Agak G. et al. J. Invest. Dermatol. 2014; 134: 366-73; Greb J. et al. Nat Rev Dis Primers. 2016; 2: 1-17). In acne overactivation of the sebaceous glands leading to the increase in sebum production is a well-known feature of this disease. Sebum is formed mainly from lipids such as triglycerides (TAG), free fatty acids, wax esters, squalene, cholesterol and cholesterol esters. Human sebum is formed mainly from lipids derived from fatty acids such as TAGs and wax esters (Pappas A. Dermatoendocrinol. 2009; 1: 72-6) and it has been shown that in humans most of the sebum is produced from de novo synthesis of fatty acids, process that is dependent of ACC activity (Esler W. P et al. WO2015/036892). Both T cells and IL-17 are increased in acne lesions and Th17 cells depend of ACC-mediated fatty acid synthesis for several functions such as the activity of the Th17 master gene RORγt and the production of pro-inflammatory cytokines such as IL-17 (Stokinger B. and Omenetti S. Nat. Rev. Immunol. 2017; 17: 535-44). Current acne treatments can be classified between topical and systemic. Topical therapies include retinoids such as adapalene, tretinoin and tazarotene, benzoyl peroxide (BPO) and antibiotics. BPO and retinoids induce skin irritation which can compromise both treatment adherence and efficacy. Topical antibiotics have limited efficacy and are associated to antibiotic resistance. The most efficacious systemic treatments are oral isotretinoin and oral antibiotics (Savage L. and Layton A. Expert Rev Clin Pharmacol. 2010; 13: 563-80). Oral isotretinoin treatment is linked to severe side effects including teratogenesis and alteration of blood lipids among others (Layton A. Dermatoendocrinol. 2009; 1: 162-9) and oral antibiotics can induce antibiotic resistance. Genetic and pharmacological evidences have shown that ACC inhibitors are useful to reduce sebum production and block IL-17 expression. However no ACC inhibitor has been approved for dermatological indications yet and the only ACC inhibitor that has progressed into clinical trials for the treatment of a dermatologic indication (Olumacostat Glasaretil for acne) has been discontinued due to lack of efficacy in a phase III study with acne patients.

In view of the numerous conditions that are contemplated to benefit from treatment involving modulation of the ACC pathway or of the AC carboxylase it is immediately apparent that compounds that modulate ACC pathways and use of these compounds should provide substantial therapeutic benefits to a wide variety of patients.

WO 2019/115405 A1 and WO2020/245297 A1 identify pyrrole compounds that act as potent ACC inhibitors. There is, however, a need to develop suitable formulations for delivery of the active agent to those in need of treatment involving modulation of the ACC pathway.

One option available is the use of a topical gel formulation for application of the active agent. There are, however, many challenges associated with the production of a topical formulation suitable for pharmaceutical use. The solubility of the active pharmaceutical ingredient can be a key issue, and problems may arise when trying to achieve adequate dissolution of the active pharmaceutical ingredient in the formulation.

An alternative approach is to use gel formulations in which the active pharmaceutical ingredient is suspended in the gel. Agglomeration of particles can, however, be a significant issue with such suspensions. High levels of agglomeration will lead to an unacceptable variation in the level of the active pharmaceutical ingredient within individual doses of the gel formulation. Furthermore, agglomeration of particles over time can lead to the formulation having an unacceptably short shelf life.

### Summary of the invention

The present invention is directed to new formulations comprising a pyrrole derivative having ACC inhibitory activity. In particular, the present invention is based on the finding that a composition comprising an ACC inhibitor with the appropriate sensory properties for application to a patient's skin (e.g., easy to apply and extend around the area of application, and not greasy) can be formulated as a gel. The gel has a long shelf life, good texture properties, and achieves acceptable skin penetration and pharmacokinetic properties.

Accordingly, the present invention provides a topical gel composition comprising: (a) a pyrrole compound, which pyrrole compound is a compound of Formula (I) or a pharmaceutically acceptable salt thereof: 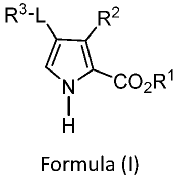 wherein: R¹ is selected from the group consisting of a hydrogen atom and a linear or branched C₁₋₄ alkyl group; R² represents a hydrogen atom or a halogen atom; L is selected from the group consisting of a direct bond, a C₅₋₆ cycloalkyl group and a phenyl ring, wherein the cycloalkyl group and the phenyl ring are unsubstituted or substituted by one or more substituents selected from a halogen atom and a linear or branched C₁₋₄ alkyl group, and R³ represents a linear or branched C₇₋₁₆ alkyl group; (b) water, and (c) a gelling agent.

### Description of the figures

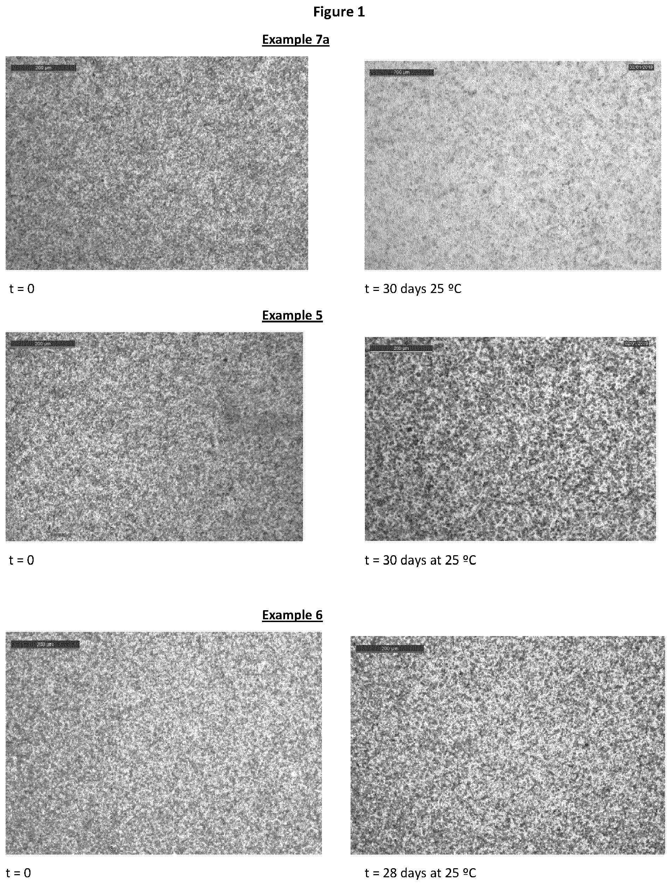

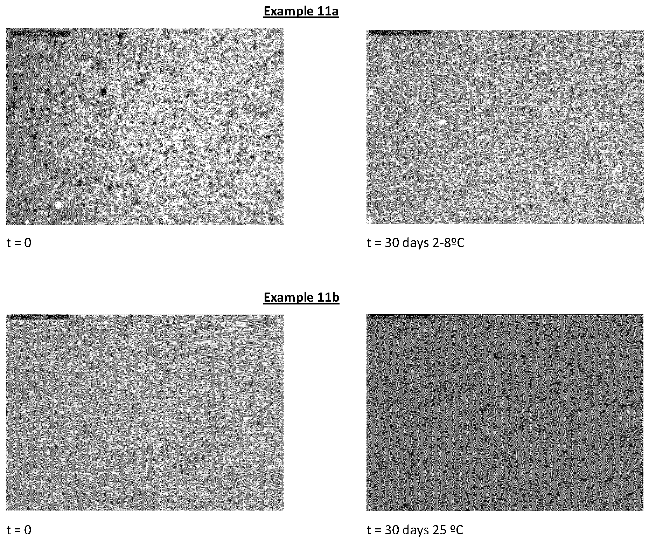

Figure 1 -The morphology of gels of the invention was determined by microscopy (Nikon Eclipse 50i).

### Detailed description of the invention

As used herein the term linear or branched C₁₋₄ alkyl group embraces unsubstituted or substituted, linear or branched radicals having 1 to 4 carbon atoms. Analogously, the term C₁₋₃ alkyl embraces linear or branched radicals having 1 to 3 carbon atoms and the term C₁₋₂ alkyl embraces linear or branched radicals having 1 to 2 carbon atoms. Analogously, the term C₂₋₄ alkyl embraces linear or branched radicals having 2 to 4 carbon atoms. Examples of C₁₋₄ alkyl include methyl, ethyl, n-propyl, *i*-propyl, n-butyl, *i*-butyl, *sec-*butyl or t-butyl. Examples of C₁₋₂ alkyl include methyl and ethyl. Such alkyl radical is typically unsubstituted or substituted by 1, 2 or 3 substituents which may be the same or different. Substituents may be selected from halogen atoms, -OH and unsubstituted -OC₁₋₃ alkyl groups. Usually such alkyl radical is unsubstituted.

As used herein the term linear or branched C₇₋₁₆ alkyl group embraces linear or branched radicals having 7 to 16 carbon atoms. Analogously, the term C₁₂₋₁₃ alkyl embraces linear or branched radicals having 12 to 13 carbon atoms. Examples of C₇₋₁₆ alkyl include heptyl, 2-ethylhexyl, octyl, nonyl, decyl, undecyl, dodecyl, tridecyl, tetradecyl, pentadecyl and hexadecyl radicals. Such alkyl radical is typically unsubstituted or substituted by 1, 2 or 3 substituents which may be the same or different. Substituents may be selected from halogen atoms, -OH and unsubstituted -OC₁₋₃ alkyl groups. Usually such alkyl radical is unsubstituted.

As used herein the term halogen atom embraces fluorine, chlorine, bromine and iodine. A halogen atom is typically a fluorine, chlorine or bromine atom, for example a fluorine or chlorine atom. The term halo when used as a prefix has the same meaning.

As used herein the term C₅₋₆cycloalkyl group embraces saturated monocyclic carbocyclic radicals having from 5 or 6 carbon atoms. Examples of monocyclic C₅₋₆ cycloalkyl groups include cyclopentyl and cyclohexyl. Such C₅₋₆ cycloalkyl radical is typically unsubstituted or substituted by 1, 2 or 3 substituents which may be the same or different. Typically, the substituents on a substituted C₅₋₆ cycloalkyl radical are selected from a halogen atom and an unsubstituted C₁₋₂ alkyl group.

When present, a phenyl ring is typically unsubstituted or substituted by 1, 2 or 3 substituents selected from a halogen atom and a linear or branched C₁₋₄ alkyl group, for example, 1, 2 or 3 substituents selected from a halogen atom and an unsubstituted linear or branched C₁₋₄ alkyl group. Preferably, the substituents on a substituted phenyl ring are selected from fluorine, chlorine and an unsubstituted C₁₋₂ alkyl group, more preferably from chorine and ethyl.

Compounds containing one or more chiral center may be used in enantiomerically or diastereomerically pure form, in the form of a racemic mixtures and in the form of mixtures enriched in one or more stereoisomer. The scope of the invention as described and claimed encompasses the racemic forms of the compounds as well as the individual enantiomers, diastereomers and stereoisomer-enriched mixtures.

Conventional techniques for the preparation/isolation of individual enantiomers include chiral synthesis from a suitable optically pure precursor or resolution of the racemate using, for example, chiral high pressure liquid chromatography (HPLC). Alternatively, the racemate (or a racemic precursor) may be reacted with a suitable optically active compound, for example, an alcohol, or, in the case where the compound contains an acidic or basic moiety, an acid or base such as tartaric acid or 1-phenethylamine. The resulting diastereomeric mixture may be separated by chromatography and/or fractional crystallization and one or both of the diastereoisomers converted to the corresponding pure enantiomer(s) by means well known to one skilled in the art. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20% and from 0 to 5% of an alkylamine, typically 0.1% diethylamine. Concentration of the eluate affords the enriched mixture. Stereoisomer conglomerates may be separated by conventional techniques known to those skilled in the art. See, e.g. "Stereochemistry of Organic Compounds" by Ernest L. Eliel (Wiley, New York, 1994).

The term "therapeutically effective amount" refers to an amount sufficient to effect treatment when administered to a patient in need of treatment.

The term "treatment" as used herein refers to the treatment of a disease or medical condition in a human patient which includes: (a) preventing the disease or medical condition from occurring, i.e., prophylactic treatment of a patient; (b) ameliorating the disease or medical condition, i.e., causing regression of the disease or medical condition in a patient; (c) suppressing the disease or medical condition, i.e., slowing the development of the disease or medical condition in a patient; or (d) alleviating the symptoms of the disease or medical condition in a patient.

The phrase "pathological condition or disease susceptible to amelioration by inhibition of ACC" includes all disease states and/or conditions that are acknowledged now, or that are found in the future, to be associated with an increased ACC activity. Such disease states include, but are not limited to, dermatological diseases, inflammatory or autoimmune-mediated diseases and a metabolism/endocrine function disorders.

As used herein, the term "pharmaceutically acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Pharmaceutically acceptable acids include both inorganic acids such as hydrochloric, sulphuric, phosphoric, diphosphoric, hydrobromic, hydroiodic or nitric acid and organic acids such as citric, fumaric, maleic, malic, ascorbic, succinic, tartaric, benzoic, acetic, methanesulphonic, ethanesulphonic, benzenesulphonic, p-toluenesulphonic acid, formic, acetic, propionic, glycolic, lactic, pyruvic, oxalic, salicylic, trichloroacetic, picric, trifluoroacetic, cinnamic, pamoic, malonic, mandelic, bismethylene salicylic, ethanedisulfonic, gluconic, citraconic, aspartic, stearic, palmitic, EDTA, p-aminobenzoic or glutamic acid, sulfates, nitrates, phosphates, perchlorates, borates, acetates, benzoates, hydroxynaphthoates, glycerophosphates or ketoglutarates. Further examples of pharmaceutically acceptable inorganic or organic acid salts include the pharmaceutically acceptable salts listed in Journal of Pharmaceutical Science, 66, 2 (1977) which are known to the skilled artisan.

Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines, lysine, guanidine, diethanolamine and choline.

It is understood by those skilled in the art that the pyrrole compounds, including the salts thereof may exist in different crystalline or polymorphic forms, all of which are intended to be within the scope of the present invention.

Typically, R¹ is selected from a hydrogen atom and an unsubstituted linear or branched C₁₋₄ alkyl group, for example, a hydrogen atom and an unsubstituted C₁₋₂ alkyl group. Preferably R¹ represents a hydrogen atom.

Typically, R² represents a halogen atom. The halogen atom may, for example, be a fluorine, chlorine or bromine atom. Preferably, R² represents a fluorine or chlorine atom.

Typically, L is selected from the group consisting of a direct bond, an unsubstituted C₅₋₆ cycloalkyl group and a phenyl ring, wherein the phenyl ring is unsubstituted or substituted by one or two substituents selected from a halogen atom and a linear or branched C₁₋₄ alkyl group. Preferably, the linear or branched C₁₋₄ alkyl substituent is itself unsubstituted.

The unsubstituted C₅₋₆ cycloalkyl group may be a cyclopentyl or a cyclohexyl group. Preferably the unsubstituted C₅₋₆ cycloalkyl group is a cyclohexyl group.

When the phenyl ring is substituted it is typically substituted by one substituent selected from a halogen atom and a linear or branched C₁₋₄ alkyl group.

Preferably L is selected from the group consisting of a direct bond, an unsubstituted C₅₋₆ cycloalkyl group (such as cyclohexyl) and a phenyl ring, wherein the phenyl ring is unsubstituted or substituted by one or two substituents selected from a halogen atom and an unsubstituted C₁₋₂ alkyl group.

More preferably, L is selected from the group consisting of a direct bond and a phenyl ring, wherein the phenyl ring is unsubstituted or substituted by one or two substituents selected from a halogen atom and an unsubstituted C₁₋₂ alkyl group.

Typically, R³ represents a linear or branched C₇₋₁₃ alkyl group. Preferably R³ represents an unsubstituted linear or branched C₁₂₋₁₃ alkyl group.

In one embodiment, in the pyrrole compound:
- R¹ is selected from a hydrogen atom and an unsubstituted C₁₋₂ alkyl group;
- R² represents a halogen atom;
- L is selected from the group consisting of a direct bond, an unsubstituted C₅₋₆ cycloalkyl group and a phenyl ring, wherein the phenyl ring is unsubstituted or substituted by one or more substituents selected from a halogen atom and a linear or branched C₁₋₄ alkyl group; and
- R³ represents an unsubstituted linear or branched C₇₋₁₆ alkyl group.

Typically, in the pyrrole compound:
- R¹ is a hydrogen atom;
- R² represents a fluorine or chlorine atom;
- L is selected from the group consisting of a direct bond, an unsubstituted C₅₋₆ cycloalkyl group and a phenyl ring, wherein the phenyl ring is unsubstituted or substituted by one or two substituents selected from a halogen atom and an unsubstituted C₁₋₂ alkyl group; and
- R³ represents an unsubstituted linear or branched C₇₋₁₆ alkyl group.

In one particular preferred embodiment, in the pyrrole compound:
- R¹ is a hydrogen atom;
- R² represents a fluorine or chlorine atom;
- L is selected from the group consisting of a direct bond, and a phenyl ring, wherein the phenyl ring is unsubstituted or substituted by one or two substituents selected from a halogen atom and an unsubstituted C₁₋₂ alkyl group; and
- R³ represents an unsubstituted linear or branched C₁₂₋₁₃ alkyl group.

The pyrrole compound may, for example, be:
- 4-(2-Chloro-4-heptylphenyl)-3-fluoro-1H-pyrrole-2-carboxylic acid;
- 4-(2-Ethyl-4-heptylphenyl)-3-fluoro-1H-pyrrole-2-carboxylic acid;
- 3-Fluoro-4-tridecyl-1H-pyrrole-2-carboxylic acid; or
- 3- Fluoro-4-((1*r*,4s)-4-octylcyclohexyl)-1H-pyrrole-2-carboxylic acid;
or a pharmaceutically acceptable salt thereof.

Preferably the pyrrole compound is 3-Fluoro-4-tridecyl-1H-pyrrole-2-carboxylic acid, or a pharmaceutically acceptable salt thereof.

The topical gel of the invention may comprise the pyrrole compound together with one or more other active pharmaceutical ingredient. Alternatively, the pyrrole compound may be the only active pharmaceutical ingredient in the topical gel of the invention.

In the topical gel of the invention the pyrrole compound is usually present in an amount of from 0.005% to 10% by weight of the total composition. The pyrrole compound may, for example, be present in an amount of from 0.1% to 10% by weight of the total composition, preferably in an amount of from 0.5% to 7.5% by weight of the total composition.

In one embodiment the pyrrole compound may be present in an amount of from 0.5% to 5.0% by weight of the total composition, for example, about 1, 2, 3, 4 or 5% by weight of the total composition.

The composition of the invention is in the form of a gel. A gel is a semi-solid preparation. The viscosity of the gel is such that the gel is suitable for application to an area of skin. Typically the gel should not run off the area of skin to which it is applied. The use of a gel composition provides a number of advantageous properties, including a non-greasy formulation that is easy to apply to and move around the skin to achieve the desired coverage. Gels are generally well tolerated by patients given the fresh feel associated with application to the skin.

The term viscosity, as used herein, refers to the dynamic viscosity (η) of the topical gel composition. The dynamic viscosity may, for example, be measured by a high performance rheometer.

The viscosity of the topical gel of the invention, at 25°C and a shear rate of 50 s⁻¹ may, for example, be from 500 to 25000 CPs. Typically, the viscosity of the topical gel of the invention, at 25°C and at a shear rate of 50 s⁻¹ may, for example, be from 1000 to 10000 CPs.

Typically the pyrrole compound is sparingly soluble in hydrophilic and/or lipophilic solvents, particularly in the hydrophilic and lipophilic solvents suitable for use as pharmaceutical excipients in topical pharmaceutical formulations.

The solubility of the pyrrole compound in water may, for example, be less than or equal to 10 mg/mL at 25°C, for example, less than or equal to 1 mg/mL at 25°C. Typically, the solubility of the pyrrole compound in water is less than or equal to 0.5 mg/mL at 25°C. More typically, the solubility of the pyrrole compound in water is less than or equal to 0.05 mg/mL at 25°C, more typically less than or equal to 0.01 mg/mL at 25°C.

The solubility of the pyrrole compound in a solvent (e.g., water) may be measured by (i) adding increasing amounts of solvent to 2 mg of pyrrole compound, (ii) stirring vigorously for 15 minutes after addition of each amount of solvent and (iii) determining the total amount of solvent added once the pyrrole compound has been completely dissolved.

When any active pharmaceutical ingredient (API) is formulated as a gel, there is a risk that individual API particles within the gel will clump together to form large agglomerates. Such agglomeration is undesirable. For example, it will lead to the active agent being applied unevenly over the area of application and may also affect the penetration of the active agent across the skin. Further, agglomeration which takes place over time could lead to large portions of the gel composition comprising insufficient amounts of the API, leading to a failure of therapy when the gel is administered.

It is an advantageous finding of the present invention that pyrrole compounds disclosed herein can be incorporated as a suspension within a gel formulation such that particles of the API are uniformly distributed through the gel, and form a product which has long term stability (including stability against agglomeration of API particles). Accordingly, in a preferred embodiment, the pyrrole compound is present in the topical gel formulation of the invention in particulate form, preferably in the form of micronized or nanonized particles, more preferably micronized particles.

In case where pyrrole compound is present in a nanonized form, the topical gel composition will be referred to as a nanosuspension gel. Alternatively, in case the pyrrole compound is present in a micronized form, the topical gel composition will be referred to as a microsuspension gel. Preferably, the formulation of the present invention will be in the form of a microsuspension gel.

Alternatively, the pyrrole compounds disclosed herein can be dissolved within the gel formulation by means of an oil-in-water emulsion.

Preferably, the pyrrole compounds disclosed herein are incorporated as a suspension of particles within the gel formulation, more preferably in the form of micronized or nanonized particles, even more preferably in the form or micronized particles.

Typically, the gel formulation of the invention contains a suspension of particles, which particles comprise the pyrrole compound described above. Preferably, said particles are particles consisting essentially of the pyrrole compound. More preferably, said particles are particles of the pyrrole compound (i.e. they consist of the pyrrole compound).

Typically, the gel formulation of the invention contains a suspension of particles which have a particle size of 50 nm to 100 µm, for example, from 100 nm to 50 µm, preferably from 1 µm to 20 µm. More preferably from 1 µm to 10 µm, even more preferably from 4 µm to 7 µm.

Good preliminary in vivo results are obtained independently of the size of the particle (both micronized and nanonized). Due to the simpler preparation of microsuspensions, micronized particles are preferred.

Typically, particle size is measured by dynamic light scattering.

More typically, particle size is measured by dynamic light scattering as defined in international standards ISO 13321 (1996) and ISO 22412 (2008). These standards detail techniques to measure the mean particle size (z-average). The z-average may, for example, be measured using a Zetasizer Nano (Malvern) or using a particle size analyzer (Helos, from Sympatec Gmbh). Typically, the particles within the gel formulation of the invention have a z-average of from 50 nm to 100 µm, for example, from 100 nm to 50 µm, preferably from 1 µm to 20 µm. More preferably from 1 µm to 10 µm, even more preferably from 4 µm to 7 µm.

Dynamic light scattering may also be used to measure the polydispersity (polydispersity index, Pdl).
The Pdl, as used herein, is a dimensionless measure of the broadness of the size distribution calculated from the cumulants analysis. The larger the Pdl value the broader the particle size distribution within the same.

Typically the Pdl as measured by dynamic light scattering, is less than 1, for example, less than 0.75. Preferably, the Pdl, as measured by dynamic light scattering, is less than 0.5.

For the avoidance of doubt, references herein to particle size are to the overall size of an aggregation of solid material within the gel vehicle. Thus, where primary particles aggregate to form agglomerates within the gel vehicle, the agglomerates will have a particle size as defined above. Preferably, there is substantially no agglomeration of particles within the gel vehicle of the invention.

In a further embodiment, the gel formulation of the invention contains both (a) pyrrole compound dissolved within the gel formulation and (b) pyrrole compound present within the gel formulation as a particulate suspension, as defined above, wherein the amount of the pyrrole compound dissolved within the gel formulation is less than or equal to 20 %, for example 10 %, e.g., 7.5 %, based on the total amount of the pyrrole compound within the gel formulation. Preferably the amount of the pyrrole compound dissolved within the gel formulation is less than or equal to 6 %, more preferably 5 %, most preferably 2.5 %, based on the total amount of the pyrrole compound within the gel formulation.

The water is usually present in an amount of at least 45 % by weight of the total composition, preferably at least 55% by weight of the total composition, more preferably at least 80 % by weight of the total composition and even more preferably at least 85 % by weight of the total composition.

As used herein the term gelling agent, or gel-forming agent, refers to a compound which, together with the water present in the topical gel of the invention, forms a viscoelastic mass. As the skilled person will appreciate, any suitable gelling agent may be used in the topical gel of the invention.

Suitable gelling agents include natural gelling agents, polymeric gelling agents, cellulose-based gelling agents and inorganic gelling agents, and mixtures thereof. Preferably, the gelling agent is a polymeric gelling agent or a cellulose-based gelling agent, more preferably a polymeric gelling agent.

Gelling agents include, but are not limited to, xanthan gum, gellan gum, guar gum, pectin, gelatin, algin (or alginic acid), carrageenan, carbomers (carbomer 934P, carbomer 940, carbomer 941), Pemulen^{™} polymeric emulsifiers, hydroxypropyl cellulose (HPC), carboxymethylcellulose (CMC), ethyl cellulose, hydroxyethyl cellulose (HEC), methyl cellulose (MC), hydroxypropyl methyl cellulose (HPMC), ethyl hydroxyethyl cellulose (EHEC), methyl hydroxyethyl cellulose(MHEC), glyceryl behenate, glyceryl monooleate, bentonite, magnesium aluminium silicate and aluminium hydroxide. Other examples include vinyl homopolymers and copolymers, for example, vinyl homopolymers and copolymers based on acrylic acid or methacrylic acid or esters thereof and methyl methacrylate, e.g., ethyl acrylate-methyl methacrylate copolymer, methacrylic acid-methylmethacrylate copolymer and butyl methacrylate-methyl methacrylate copolymer.

Examples of commercially available gelling agents suitable for use as a gelling agent in topical gel composition of the present invention are: (i) Sepineo^{™} P600, which comprises acrylamide, sodium acryloyldimethyl taurate copolymer, isohexadecane and polysorbate 80; (ii) Sepineo^{™} DERM, which comprises hydroxyethyl acrylate, sodium acryloyldimethyl taurate copolymer; (iii) Pemulen^{™} TR-2, which comprises acrylates and a C10-30 alkyl acrylate crosspolymer; and (iv) Methocel^{™} 4KM, which comprises hydroxypropyl methylcellulose.

Typically, the gelling agent is selected from the group consisting of: xanthan gum; a acrylamide/sodium acryloyldimethyl taurate copolymer/isohexadecane and polysorbate 80 mixture; hydroxyethyl acrylate/sodium acryloyldimethyl taurate copolymer; acrylates/C10-30 alkyl acrylate crosspolymer; methylcellulose; and hydroxypropyl methylcellulose. Preferably the gelling agent is a acrylamide/sodium acryloyldimethyl taurate copolymer/isohexadecane and polysorbate 80 mixture, sold under the name Sepineo^{™} P600.

Typically the gelling agent is present in an amount from 0.5 % to 5 % by weight of the total composition, preferably from 1 % to 4 % by weight of the total composition, more preferably from 2.5 to 3.5 % by weight of the total composition.

In one embodiment, the gelling agent is present in an amount of about 2, 3 or 4 % by weight of the total composition, for example, about 3 % by weight of the total composition.

The topical gel composition of the invention may further comprise: (d) a surfactant; and/or (e) a preservative system; and/or (f) a moisturizer. The surfactant, if present, may of course be present on the surfaces of the particles within the gel formulation described above.

The topical gel composition of the invention may, for example, comprise (a) a pyrrole compound; (b) water; (c) a gelling agent; and (d) a surfactant.

In some embodiments the topical gel composition of the invention comprises (a) a pyrrole compound; (b) water; (c) a gelling agent; (d) a surfactant; and (e) a preservative system.

Alternatively, the topical gel composition of the invention may comprise (a) a pyrrole compound; (b) water; (c) a gelling agent; (d) a surfactant; and (f) a moisturizer.

In a further embodiment the topical gel composition of the invention comprises (a) a pyrrole compound; (b) water; (c) a gelling agent; (d) a surfactant; (e) a preservative system; and (f) a moisturizer.

The surfactant (or emulsifier) may be any surfactant suitable for use in a pharmaceutical formulation, e.g. a non-ionic surfactant, a cationic surfactant, an anionic surfactant, a zwitterionic surfactant, or a mixture thereof. Typically, the surfactant is a non-ionic surfactant.

Examples of surfactants include lecithin, oleic acid, polyoxyethylene glycol alkyl ethers (for instance PEG 300, PEG 600, PEG 1000, Brij 30, Brij 35, Brij 56, Brij 76 and Brij 97), polypropylene glycol (for instance PPG 2000), glucoside alkyl ethers, polyoxyethylene glycol octylphenol ethers, polyoxyethylene glycol alkylphenol ethers, glycerol alkyl esters, polyoxyethylene glycol sorbitan alkyl esters (polysorbates, for instance polysorbate 20, polysorbate 40, polysorbate 60 and polysorbate 80), sorbitan alkyl esters (for instance sorbitan monolaurate (Span 20), sorbitan monooleate (Span 80) and sorbitan trioleate (Span 85)), cocamide MEA, cocamide DEA, dodecyldimethylamine oxide, block copolymers of polyethylene glycol and polypropylene glycol (poloxamers), block copolymers of polyethylene glycol and polypropylene oxide (for instance Pluronic surfactants), polyvinyl pyrrolidone K25, polyvinyl alcohol, oligolactic acid, sodium dioctyl sulfosuccinate and polyethoxylated tallow amine (POEA). Others include, sodium lauryl sulfate (sodium dodecyl sulfate, SLS, or SDS), and the related alkyl-ether sulfates such as sodium laureth sulfate (sodium lauryl ether sulfate or SLES), and sodium myreth sulfate, docusate (dioctyl sodium sulfosuccinate), perfluorooctanesulfonate (PFOS), perfluorobutanesulfonate, alkyl-aryl ether phosphates, alkyl ether phosphates, carboxylates (such as sodium stearate), sodium lauroyl sarcosinate and carboxylate-based fluorosurfactants (such as perfluorononanoate and perfluorooctanoate (PFOA or PFO)). Polyethylene glycol derivatives of the castor oil or the hydrogenated castor oil such as PEG-30 castor oil, PEG-33 castor oil, PEG-35 castor oil, PEG-36 castor oil and PEG-40 castor oil, PEG-30 hydrogenated castor oil and PEG-40 hydrogenated castor oil, PEG-80 glyceryl cocoate, derivatives thereof, and the like, or mixtures of thereof may also act as surfactants.

Examples of commerically available surfactants that may be used in the present invention include: Labrasol^{®} (which is a mixture of acylglycerols and PEG esters); Lauroglycol^{™} FCC (which comprises propylene glycol monolaurate); Tween^{™} 80 (polysorbate 80); Tween^{™} 20 (polysorbate 20); and Arlamol^{™} PS11E (polyoxypropylene stearyl ether)

Typically, the surfactant is selected from the group consisting of Labrasol^{®}, Lauroglycol^{™} FCC, Polysorbate 20, Polysorbate 80, Arlamol^{™} PS11E or a mixture thereof, preferably Arlamol^{™} PS11E and Polysorbate 80, more preferably Polysorbate 80.
When present in the topical gel composition of the invention, typically, the surfactant is present in an amount of from 0 % to 7 % by weight of the total composition, preferably from 0.2 % to 5 % by weight of the total composition, more preferably from 0.5 % to 3.5 % by weight of the total composition.

The preservative system comprises one or more preservatives, for example, one, two, three or four preservatives. Usually the preservative system comprises up to three preservatives, e.g., one, two or three preservatives.

The preservative may, for example, be any pharmaceutically acceptable preservative. Examples of suitable preservatives are alkylparabens (particularly methylparaben, ethylparaben, propylparaben and butylparaben), sodium benzoate, butylated hydroxy toluene, butylated hydroxyanisole, ethylenediamine tetraacetic acid, chlorobutanol, benzyl alcohol, phenylethylalcohol, phenoxyethanol, dehydroacetic acid, sorbic acid, undecylenic acid, potassium sorbate, benzalkonium chloride, benzethonium chloride, and mixtures thereof. The amount of preservative generally utilized will typically vary depending upon the preservative selected.

Typically, the preservative is selected from the group consisting of phenoxyethanol, EDTA, Salinip XB (a mixture of phenoxyethanol, methyl paraben, propyl paraben, ethyl paraben), methylparaben sodium, propylparaben sodium, benzyl alcohol, nipagin (methylparaben) and nipasol (propylparaben) or a mixture thereof. One particularly preferred mixture of preservatives is methylparaben, propylparaben and phenoxyethanol.

When present in the topical gel composition of the invention, typically, the preservative is present in an amount of from 0.1 % to 5 % by weight of the total composition, preferably in an amount of from 0.2 % to 2.5 % by weight of the total composition, more preferably in an amount of from 0.5 % to 1.5 % by weight of the total composition.

In one embodiment, the preservative is present in an amount of about 0.5, 1, 1.22 or 1.5 % by weight of the total composition, for example, in an amount of about 1.22 % by weight of the total composition.

The moisturizer may be any moisturizer suitable for use in a pharmaceutical composition. Typically, the moisturizer is selected from the group consisting of an emollient, an occlusive, a humectant and a mixture thereof.

The moisturizer may, for example, be selected from the group consisting of fatty acids, fatty alcohols, cholesterol, squalene, pseudoceramides, urea, sorbitol, glycerol, propylene glycol, hyaluronic acid, alpha hydroxy acids, mineral oil, petroleum jelly, beeswax, silicones and zinc oxide. Examples also include isopropyl palmitate, isostearyl alcohol, decyl oleate, propylene glycol, octyl stearate, glyceryl stearate, jojoba oil, castor oil, dimethicone, cyclomethicone, octyl octanoate, isopropyl myristate, Isopropyl isostearate, diisopropyl dilinoleate, sorbitol, glycerin, panthenol, gelatin, glycolic acid, lactic acid, sodium pyrrolidine, butylene glycol, paraffin, mineral oil, caprylic/capric triglyceride, stearic acid, lanolin acid, lanolin, cetyl alcohol, stearyl alcohol, lecithin, candelilla, carnauba, lanolin and stearyl stearate.

Typically, the moisturizer is selected from the group consisting of diisopropyl adipate, octyldodecanol (Kollicream^{®} OD), glycerides, mixed decanoyl and octanoyl (Kollisolv^{®} MCT 70), liquid vaseline, propylene glycol and glycerine, and a mixture thereof.

When present in the topical gel composition of the invention, typically the moisturizer is present in an amount of from 20 % to 45 % by weight of the total composition, preferably from 30 % to 40 % by weight of the total composition.

Typically, when the moisturizer is present in an amount of from 20 % to 45 % by weight of the total composition, the water is present in an amount of at least 45 % by weight of the total composition. Preferably, when the moisturizer is present in an amount of from 30 % to 40 % by weight of the total composition the water is present in an amount of at least 47 % by weight of the total composition.

The stability of a pharmaceutical gel composition is a factor in patient compliance, as well as the safety and efficacy of the product. Texture analysis may be used to assess the characteristics of a gel formulation during production and over the longer term. The topical gel composition of the invention exhibits hardness and adhesiveness values making it particularly advantageous for use in topical treatments to the skin.
The gel strength (or hardness) of the topical gel composition of the invention may be measured by measuring the mass, in grams, necessary to depress a standard plunger 4 mm into the gel, e.g., using a TA.XT 2 Stable Micro System's texture analyser.

Typically, the hardness of the topical gel composition of the invention is less than or equal to 20 g, for example less than or equal to 15 g. Preferably, the hardness of the topical gel composition of the invention is less than or equal to 13.0 g.

For example, the hardness of the topical gel composition of the invention may be from 0 to 20 g. Typically, the hardness of the topical gel composition of the invention is from 1 to 15 g. Preferably, the hardness of the topical gel composition of the invention is from 5.0 to 13.0 g, more preferably from 8.0 to 11.0 g.

Reversal of the gel strength test may also be used to assess the adhesive properties of the topical gel composition of the invention. The adhesiveness provides a measure of the energy required to separate the probe from the sample on the return stroke.

Typically, the adhesiveness of the topical gel composition of the invention is from 0 to -80 g.sec, for example, from -5 to -60 g.sec.

In a preferred embodiment, the topical gel composition of the invention comprises:
(a) a pyrrole compound as defined above;
(b) water;
(c) a gelling agent selected from the group consisting of: xanthan gum; a acrylamide/sodium acryloyldimethyl taurate copolymer/isohexadecane and polysorbate 80 mixture; hydroxyethyl acrylate/sodium acryloyldimethyl taurate copolymer; acrylates/C10-30 alkyl acrylate crosspolymer; methylcellulose; and hydroxypropyl methylcellulose; and optionally
(d) a surfactant.

For example, the topical gel composition of the invention comprises:
(a) a pyrrole compound as defined above;
(b) water;
(c) a gelling agent; and
(d) a surfactant selected from the group consisting of Labrasol, Lauroglycol^{™} FCC, Polysorbate 20, Polysorbate 80, Arlamol^{™} PS11E or a mixture thereof.

In another example, the topical gel composition of the invention comprises:
(e) a pyrrole compound as defined above;
(f) water;
(g) a gelling agent selected from the group consisting of: xanthan gum; a acrylamide/sodium acryloyldimethyl taurate copolymer/isohexadecane and polysorbate 80 mixture; hydroxyethyl acrylate/sodium acryloyldimethyl taurate copolymer; acrylates/C10-30 alkyl acrylate crosspolymer; methylcellulose; and hydroxypropyl methylcellulose; and
(h) a surfactant selected from the group consisting of Labrasol, Lauroglycol^{™} FCC, Polysorbate 20, Polysorbate 80, Arlamol^{™} PS11E or a mixture thereof.

In one embodiment, the topical gel composition of the invention comprises:
(a) 3-Fluoro-4-tridecyl-1H-pyrrole-2-carboxylic acid or a pharmaceutically acceptable salt thereof;
(b) water;
(c) a gelling agent; and optionally
(d) a surfactant.

In another embodiment, the topical gel composition of the invention comprises:
(a) 3-Fluoro-4-tridecyl-1H-pyrrole-2-carboxylic acid or a pharmaceutically acceptable salt thereof;
(b) water;
(c) a gelling agent; and optionally
(d) a surfactant selected from the group consisting of Labrasol^{®}, Lauroglycol^{™} FCC, Polysorbate 20, Polysorbate 80, Arlamol^{™} PS11E or a mixture thereof.

In another embodiment, the topical gel composition of the invention comprises:
(a) 3-Fluoro-4-tridecyl-1H-pyrrole-2-carboxylic acid or a pharmaceutically acceptable salt thereof;
(b) water;
(c) a gelling agent selected from the group consisting of: xanthan gum; a acrylamide/sodium acryloyldimethyl taurate copolymer/isohexadecane and polysorbate 80 mixture; hydroxyethyl acrylate/sodium acryloyldimethyl taurate copolymer; acrylates/C10-30 alkyl acrylate crosspolymer; methylcellulose; and hydroxypropyl methylcellulose ; and optionally
(d) a surfactant.

In another preferred embodiment, the topical gel composition of the invention comprises:
(a) 3-Fluoro-4-tridecyl-1H-pyrrole-2-carboxylic acid or a pharmaceutically acceptable salt thereof;
(b) water;
(c) a gelling agent selected from the group consisting of: xanthan gum; a acrylamide/sodium acryloyldimethyl taurate copolymer/isohexadecane and polysorbate 80 mixture; hydroxyethyl acrylate/sodium acryloyldimethyl taurate copolymer; acrylates/C10-30 alkyl acrylate crosspolymer; methylcellulose; and hydroxypropyl methylcellulose ; and optionally
(d) a surfactant selected from the group consisting of Labrasol^{®}, Lauroglycol^{™} FCC, Polysorbate 20, Polysorbate 80, Arlamol^{™} PS11E or a mixture thereof.

For example, the topical gel composition of the invention comprises:
(a) 3-Fluoro-4-tridecyl-1H-pyrrole-2-carboxylic acid or a pharmaceutically acceptable salt thereof;
(b) water;
(c) Sepineo^{™} P600; and
(d) Polysorbate 80.

In a preferred embodiment, the topical gel composition of the invention comprises:
(a) 3-Fluoro-4-tridecyl-1H-pyrrole-2-carboxylic acid, or a pharmaceutically acceptable salt thereof, in an amount of from 0.5% to 7.5 % by weight of the total composition;
(b) water in an amount at least 85% by weight of the total composition;
(c) Sepineo^{™} P600 in an amount of from 2.5 to 3.5 % by weight of the total composition; and
(d) Polysorbate 80 in an amount of from 0.5% to 3% by weight of the total composition.

The topical gel composition of the invention may, for example, comprise:
(a) 3-Fluoro-4-tridecyl-1H-pyrrole-2-carboxylic acid, or a pharmaceutically acceptable salt thereof, in an amount of from 0.5% to 7.5 % by weight of the total composition;
(b) water in an amount at least 85% by weight of the total composition;
(c) Sepineo^{™} P600 in an amount of from 2.5 to 3.5 % by weight of the total composition;
(d) Polysorbate 80 in an amount of from 0.5 % to 3% by weight of the total composition; and
(e) a preservative system comprising methylparaben, propylparaben and phenoxyethanol,
wherein the preservative system is in an amount of from 0.5% to 1.5% by weight of the total composition.

In another embodiment, the topical gel composition of the invention comprises:
(a) 3-Fluoro-4-((1r,4s)-4-octylcyclohexyl) -1H-pyrrole-2-carboxylic acid or a pharmaceutically acceptable salt thereof;
(b) water;
(c) Sepineo^{™} P600; and
(d) Polysorbate 80

For example, the topical gel composition of the invention comprises:
(a) 3-Fluoro-4-((1r,4s)-4-octylcyclohexyl) -1H-pyrrole-2-carboxylic acid, or a pharmaceutically acceptable salt thereof, in an amount of from 0.5% to 7.5 % by weight of the total composition;
(b) water in an amount at least 85% by weight of the total composition;
(c) Sepineo^{™} P600 in an amount of from 2.5 to 3.5% by weight of the total composition; and
(d) Polysorbate 80 in an amount of from 0.2 % to 5% by weight of the total composition.

In a further embodiment, the topical gel composition of the invention comprises:
(a) a pyrrole compound as defined above;
(b) water;
(c) a gelling agent selected from the group consisting of: xanthan gum; a acrylamide/sodium acryloyldimethyl taurate copolymer/isohexadecane and polysorbate 80 mixture; hydroxyethyl acrylate/sodium acryloyldimethyl taurate copolymer; acrylates/C10-30 alkyl acrylate crosspolymer; methylcellulose; and hydroxypropyl methylcellulose; and
(d) a surfactant selected from the group consisting of Labrasol^{®}, Lauroglycol^{™} FCC, Polysorbate 20, Polysorbate 80, Arlamol^{™} PS11E or a mixture thereof; and optionally
(e) a moisturizer selected from the group consisting of diisopropyl adipate, Kollisolv^{®} MCT 70, Kollicream^{®} OD, liquid Vaseline or a mixture thereof.

The topical gel composition of the invention may, for example, comprise:
(a) 4-(2-Chloro-4-heptylphenyl)-3-fluoro-1H-pyrrole-2-carboxylic acid, or a pharmaceutically acceptable salt thereof, in an amount of from 0.5% to 7.5 % by weight of the total composition;
(b) water in an amount at least 45% by weight of the total composition;
(c) Sepineo^{™} P600 in an amount of from 2.5 to 3.5 % by weight of the total composition; and
(d) a surfactant system comprising Tween^{™} 80 and Lauroglycol^{™} FCC in an amount of from 0 % to 7% by weight of the total composition;
(e) a moisturizer system comprising a mixture of diisopropyl adipate, Kollisolv^{®} MCT 70 and Kollicream^{®} OD in an amount of from 20% to 45% by weight of the total composition; and
(f) a preservative system comprising a mixture of methylparaben, propylparaben and phenoxyethanol, wherein the preservative system is in an amount of from 0.5% to 1.5% by weight of the total composition

The topical gel composition of the invention may, in another exemple, comprise:
(a) 4-(2-Ethyl-4-heptylphenyl)-3-fluoro-1H-pyrrole-2-carboxylic acid, or a pharmaceutically acceptable salt thereof, in an amount of from 0.5% to 7.5 % by weight of the total composition
(b) water in an amount of at least 45% by weight of the total composition;
(c) Sepineo^{™} P600 in an amount of from 0.5% to 5.0% by weight of the total composition; and
(d) a surfactant system comprising a mixture of Labrasol^{®} and Lauroglycol^{™} FCC in an amount of from 0% to 7% by weight of the total composition;
(e) a moisturizer system comprising a mixture of Diisopropyl adipate, Kollisolv^{®} MCT 70, Kollicream^{®} OD and Glycerin in an amount of from 20% to 45% by weight of the total composition; and
(f) benzyl alcohol in an amount of from 0.5% to 1.5% by weight of the total composition.

### Medical uses

The topical gel composition of the invention may be for use in the treatment or prevention of a dermatological disease.

Dermatological diseases include acne vulgaris, acne conglobata, inflammatory acne, choracne, rosacea, Rhinophyma-type rosacea, seborrhea, seborrheic dermatitis, sebaceous gland hyperplasia, Meibomian gland dysfunction of facial rosacea, mitogenic alopecia, oily skin, plaque psoriasis, guttate psoriasis, inverse psoriasis, erythrodermic psoriasis, scalp psoriasis, nail psoriasis, postular psoriasis and palmoplantar pustulosis; preferably in the treatment of acne vulgaris, acne conglobata, inflammatory acne, choracne, plaque psoriasis, guttate psoriasis, inverse psoriasis, erythrodermic psoriasis, scalp psoriasis, nail psoriasis and pustular psoriasis.

Typically, the topical gel composition of the invention is for use in the treatment of acne vulgaris, acne conglobata, inflammatory acne, choracne, plaque psoriasis, guttate psoriasis, inverse psoriasis, erythrodermic psoriasis, scalp psoriasis or pustular psoriasis. Preferably, the topical gel composition of the invention is for use in the treatment of acne vulgaris.

The invention also provides a method of preventing or treating a dermatological disease using the topical gel composition of the invention, which method comprises applying a therapeutically effective amount of the topical gel composition to the skin of a patient. The dermatological disease may, for example, be as described herein.

Also provided is the use of the topical gel composition of the invention in the manufacture of a medicament for use in preventing or treating a dermatological disease. The dermatological disease may, for example, be as described herein.

The topical gel composition of the invention may comprise, or be used in combination with, a second active compound in preventing or treating a dermatological disease.

### General synthetic procedure

The pyrrole compounds may be prepared using the methods and procedures described herein, or using similar methods and procedures. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Starting compounds are commercially available or may be obtained following the conventional synthetic methods already known in the art.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein.

WO 2019/115405 A1 and WO2020/245297 A1 provide illustrations of specific synthetic processes that may be used to obtain compounds of formula (I). The content of both WO 2019/115405 A1 and WO2020/245297 A1 is incorporated herein by reference.

According to one embodiment of the present invention, compounds of general formula (I') and (I"), subsets of general formula (I), wherein R¹-R³ and L are as defined in the claims, may be prepared by the following synthetic route as illustrated in Scheme 1:

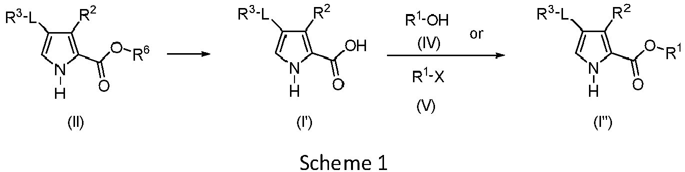

Compounds of general formula (I"), a subset of general formula (I), wherein R¹ is other than a hydrogen atom, may be obtained from compounds of general formula (I'), a subset of general formula (I), wherein R¹ is a hydrogen atom, by reaction with alcohols of formula (IV) in the presence of a base such as 4-dimethylaminopyridine or triethylamine and a coupling reagent such as 3-((ethylimino) methyleneamino)-*N,N*-dimethylpropan-1-aminium chloride (EDCI-HCI) or dicyclohexylcarbodiimide (DCC), in a solvent such as methylene chloride at room temperature. Compounds of formula (I") may also be prepared from compounds of formula (I') following a different synthetic approach. Reaction of compounds of formula (I') with a suitable chlorinating reagent such as oxalyl chloride in the presence of a catalytic amount of *N,N-*dimethylformamide in a solvent such as methylene chloride at room temperature gives rise to intermediate acid chlorides which may be treated with alcohols of general formula (IV) without the presence of a base or in the presence of a base such as triethylamine, without the use of a solvent or in a solvent such as methylene chloride at temperatures ranging from 0 °C to room temperature to provide compounds of formula (I"). Alternatively, compounds of formula (I") may also be obtained by reaction of compounds of formula (I') with haloderivatives of formula (V), wherein X represents a halogen atom, in the presence of a base such as potassium carbonate or triethylamine, in a solvent such as acetonitrile or *N,N*-dimethylformamide at temperatures ranging from room temperature to reflux.

In a particular case, compounds of formula (I"), in which the residue at R¹ contains an alcohol or diol moiety functionalized with an appropriate protecting group such as benzyl (Bn) or benzylidene acetal, may be deprotected at the alcohol or diol moiety under standard conditions (Greene's Protective Groups in Organic Synthesis, ISBN: 0471697540).

Compounds of formula (I'), a subset of formula (I), wherein R¹ is a hydrogen atom, may be obtained from compounds of formula (II). Compounds of formula (II), wherein R⁶ represents an alkyl group such as methyl or ethyl group, may be treated with a suitable base such as lithium hydroxide, sodium hydroxide or potassium hydroxide, in a solvent such as methanol, ethanol or tetrahydrofuran, with or without the presence of water as co-solvent, at temperatures ranging from ambient temperature to reflux, to furnish compounds of formula (I').

In a particular case, compounds of general formula (IIa) (see Scheme 2) wherein L is a direct bond, R⁷ represents a linear or branched C₇₋₁₆ alkyl group which may be substituted by one or more halogen atoms and R² is as defined in the claims, may be prepared by the following synthetic route as illustrated in Scheme 2:

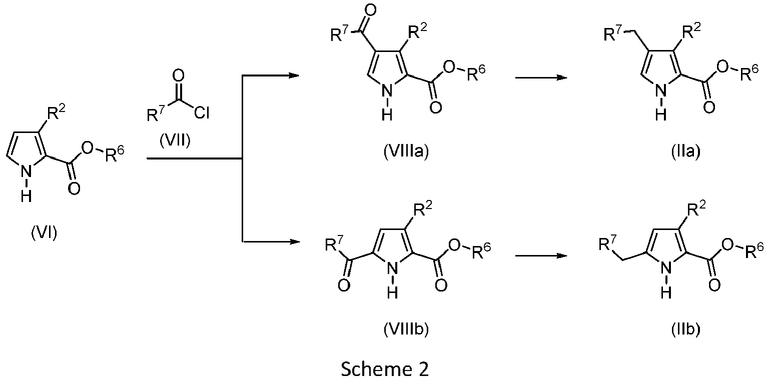

Pyrroles of formula (VI) may be reacted with acid chlorides of formula (VII) in the presence of a Lewis acid such as zinc(II) chloride, aluminium(III) chloride, tin(IV) chloride or boron trifluoride diethyl etherate, in a solvent such as methylene chloride, 1,2-dichloroethane or benzene, at temperatures ranging from 0 ºC to room temperature, to furnish ketones of formulas (VIIIa) and (Vlllb). The ratio among regioisomers (VIIIa) and (Vlllb) may vary depending on the Lewis acid and the reaction conditions used. Reduction of ketones of formulas (VIIIa) and (Vlllb) by treatment with triethylsilane and trifluoroacetic acid, with or without the use of a Lewis acid such as boron trifluoride diethyl etherate, at room temperature, furnishes compounds of formulas (IIa) and (IIb) respectively.

In another particular case, compounds of formulas (IIc) and (IId), subsets of general formula (I), wherein L is a cyclohexane ring, may be prepared by the following synthetic route as illustrated in Scheme 3:

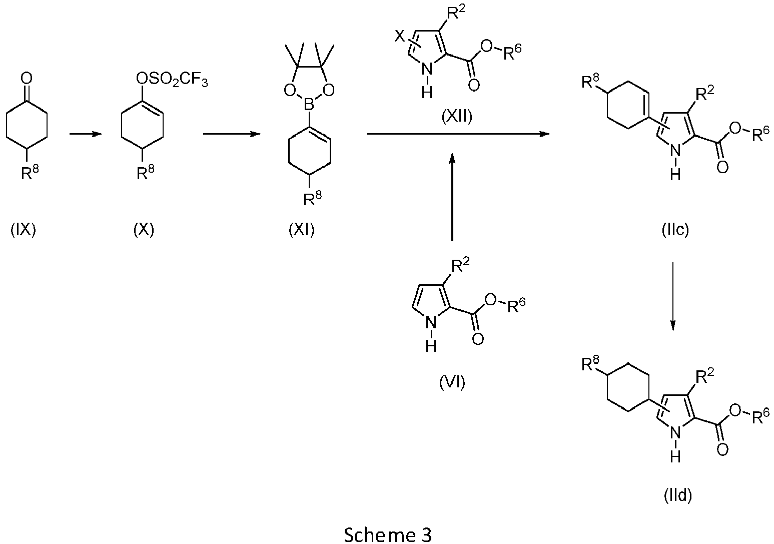

Trifluoromethanesulfonates of formula (X) may be prepared from ketones of formula (IX) by treatment with a suitable base such as lithium bis(trimethylsilyl)amide in a solvent such as tetrahydrofuran at -78 ºC followed by addition of 1,1,1-trifluoro-N-phenyl-N-((trifluoromethyl)sulfonyl)methanesulfonamide. Trifluoromethanesulfonates of formula (X) may be converted into boronates of formula (XI) by reaction with 4,4,4',4',5,5,5',5'-octamethyl-2,2'-bi(1,3,2-dioxaborolane), in the presence of a suitable palladium catalyst such as bis(triphenylphosphine)palladium(II) dichloride, and a base, such as potassium acetate, in a solvent such as 1,4-dioxane, at 90 ºC under an argon atmosphere. Reaction of boronates of formula (XI) with halogenated pyrroles of formula (XII) in the presence of a suitable palladium catalyst such as [1,1'-bis(diphenylphosphino)ferrocene]palladium(II) dichloride dichloromethane complex, in a solvent such as 1,4-dioxane, with the use of water as co-solvent, in the presence of a base such as cesium carbonate, at 100 °C, provides compounds of formula (IIc).

Reaction of pyrroles of formula (VI) with *N*-bromosuccinimide in a solvent such as methylene chloride at room temperature, or with diiodine in a solvent such as *N,N*-dimethylformamide, at temperatures ranging from 0 ºC to room temperature, furnishes halogenated pyrroles of formula (XII), wherein X is a bromine or a iodine atom respectively.

Reduction of compounds of formula (IIc) with hydrogen in the presence of palladium on carbon as catalyst, in a solvent such as methanol or ethanol at room temperature, furnishes compounds of formula (IId).

Reaction of dihalogenated phenyl derivatives of formula (XIII) with acetylenic derivatives of formula (XIV) in the presence of copper and palladium catalysts such as copper (I) iodide and tetrakis(triphenylphosphine)palladium(0), in a solvent such as a mixture of dichloromethane and trietylamine, at 60 ºC under an argon atmosphere, gives rise to compounds of formula (XV). Reduction of compounds of formula (XV) with hydrogen in the presence of platinum (IV) oxide or chloridotris(triphenylphosphane)rhodium(I) as catalysts, in a solvent such as ethanol or toluene, with or without the presence of a base, such as trimethylamine, at a pressure ranging from atmospheric pressure to 50 bar, furnishes bromoderivatives of formula (XVI).

Bromophenyl derivatives of formula (XVI) may be converted into boronates of formula (XVII) by reaction with 4,4,4',4',5,5,5',5'-octamethyl-2,2'-bi(1,3,2-dioxaborolane), in the presence of a suitable catalyst such as bis(triphenylphosphine) palladium(II) dichloride, and a base, such as potassium acetate, in a solvent such as 1,4-dioxane, at 90 ºC under an argon atmosphere.

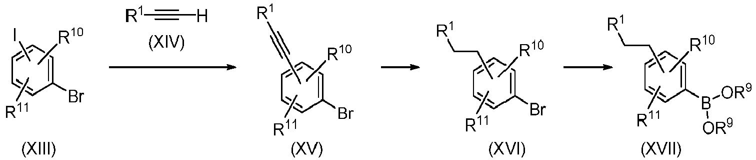

In another particular case, compounds of formula (IIe), a subset of general formula (I), wherein L is a phenyl ring, may be prepared by the following synthetic route as illustrated in Scheme 4:

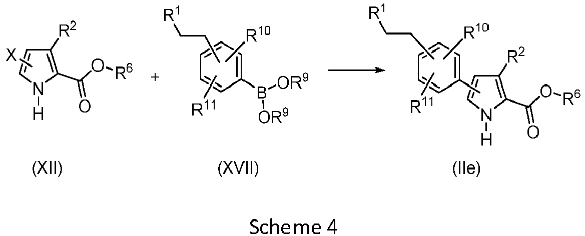

Halogenated pyrroles of formula (XII) may be reacted with boronic acids (wherein R⁹ is a hydrogen atom) or boronate esters (wherein R⁹ is an alkyl group) of formula (XVII) under Suzuki-Miyaura reaction conditions (Miyaura, N.; Suzuki, A. Chem. Rev. 1995, 95, 2457) to give compounds of formula (IIe). Such reactions may be catalyzed by a suitable palladium catalyst such as [1,1'-bis(diphenylphosphino)ferrocene]palladium(II) dichloride dichloromethane complex or tetrakis(triphenylphosphine)palladium(0), in a solvent such as toluene or 1,4-dioxane, with or without the use of water as co-solvent, in the presence of a base such as cesium carbonate or sodium carbonate, at temperatures ranging from 80 ºC to 110 °C, with or without the use of microwave irradiation.

### Example compounds of formula (I)

The general synthetic procedure is illustrated below for four example compounds of formula (I). Reagents, starting materials, and solvents were purchased from commercial suppliers and used as received. Commercial intermediates are referred to in the experimental section by their IUPAC name. Ether refers to diethyl ether, unless otherwise specified. Concentration or evaporation refer to evaporation under vacuum using a Büchi rotatory evaporator.

Reaction products were purified, when necessary, by flash chromatography on silica gel (40-63 µm) with the solvent system indicated. Purifications in reverse phase were made in a Biotage Isolera^{®} automated purification system equipped with a C18 column and using a gradient, unless otherwise stated, of water-acetonitrile/MeOH (1:1) (0.1% v/v ammonium formate both phases) from 0% to 100% acetonitrile/MeOH (1:1) in 40 column volumes. The conditions "formic acid buffer" refer to the use of 0.1% v/v formic acid in both phases. The appropriate fractions were collected and the solvents evaporated under reduced pressure and/or liofilized.

Purifications in reverse phase were also made in a Biotage SP1^{®} automated purification system equipped with a C18 column and using a gradient of, unless otherwise stated, water-acetonitrile/MeOH (1:1) (0.1% v/v ammonium formate both phases) from 0% to 100% acetonitrile/MeOH (1:1) in 80 column volumes. The conditions "formic acid buffer" refer to the use of 0.1% v/v formic acid in both phases. The appropriate fractions were collected and freeze dried.

Gas chromatography was performed using a Thermo Trace Ultra gas chromatograph, coupled to a DSQ mass detector. Injections were performed on a split/splitless injector and a HP-1MS was the capillary column. Mass spectra were obtained by electron impact ionisation at 70 eV.

Preparative HPLC-MS were performed on a Waters instrument equipped with a 2767 injector/collector, a 2525 binary gradient pump, a 2996 PDA detector, a 515 pump as a make-up pump and a ZQ4000 Mass spectrometer detector or on a Agilent 1200 Series coupled to an Agilent 6120 Mass spectrometer detector. Both systems were equipped with a Symmetry Prep C18 (19 x 300 mm, 7 µm) column or a XBridge Prep C18 (19 x 100 mm, 5 µm) column. The mobile phase was formic acid (0.4 mL), ammonia (0.1 mL), methanol (500 mL) and acetonitrile (500 mL) (B) and formic acid (0.5 mL), ammonia (0.125 mL) and water (1000 mL) (A), the specific gradients used are specified in each particular case. The flow rate was 20 ml/min.

The UPLC chromatographic separations were obtained using a Waters Acquity UPLC system coupled to a SQD mass spectrometer detector. The system was equipped with an ACQUITY UPLC BEH C-18 (2.1x50mm, 1.7 mm) column. The mobile phase was formic acid (0.4 ml), ammonia (0.1 ml), methanol (500 ml) and acetonitrile (500 ml) (B) and formic acid (0.5 ml), ammonia (0.125 ml) and water (1000 ml) (A). A gradient between 0 to 95% of B was used. The run time was 3 or 6 minutes. The injection volume was 0.5 microliter. Chromatograms were processed at 210 nM or 254 nM. Mass spectra of the chromatograms were acquired using positive and negative electrospray ionization.
1H Nuclear Magnetic Resonance Spectra were recorded on a Varian Mercury plus operating at a frequency of 400MHz or a Varian VNMRS operating at 600MHz and equipped with a cold probe for the 1H spectra. Samples were dissolved in the specified deuterated solvent. Tetramethylsilane was used as reference.

Standard synthetic methods are described the first time they are used. Compounds synthesized with similar methods are referred to only by their starting materials, without full experimental detail. Slight modifications to the general experimental methods used are permitted in these cases. Specific synthetic transformations already described in the literature are referred to only by their bibliographical reference. Other specific methods are also described in full.

### EXAMPLE 1 - 4-(2-Chloro-4-heptylphenyl)-3-fluoro-1H-pyrrole-2-carboxylic acid

### Intermediate A - Ethyl 3-fluoro-4-iodo-1H-pyrrole-2-carboxylate

To a cooled (0 °C) solution of ethyl 3-fluoro-1H-pyrrole-2-carboxylate (1.00 g, 6.36 mmol) in DMF (16 mL) was added diiodine (4.84 g, 19.07 mmol) in five portions and the mixture was stirred at room temperature for 2 h. The reaction was poured into a mixture of water (100 mL) and saturated aqueous sodium thiosulfate solution (10 mL) and extracted with diethyl ether (x3). The combined organic layers were washed with water and brine, dried over magnesium sulfate, filtered and the solvent was evaporated to dryness. Purification of the residue by flash chromatography (hexanes/diethyl ether) gave the title compound (1.10 g, 61%) as a white solid.
MS (m/z): 282 [M-1]⁻.
¹H NMR δ (400 MHz, CDCl₃): 1.37 (t, J=7.1 Hz, 3H), 4.36 (q, J=7.1 Hz, 2H), 6.82 (t, J=3.7 Hz, 1H), 8.86 (s, 1H).

### Intermediate B - Ethyl 4-(2-chloro-4-heptylphenyl)-3-fluoro-1H-pyrrole-2-carboxylate

### a) 1-Bromo-2-chloro-4-(hept-1-yn-1-yl)benzene

A Schlenk tube was charged with 1-bromo-2-chloro-4-iodobenzene, hept-1-yne, DCM and TEA. The Schlenk tube was subjected to three cycles of evacuation-backfilling with argon and then tetrakis(triphenylphosphine)palladium(0) and copper (I) iodide were added. After three further cycles of evacuation-backfilling with argon, the Schlenk tube was sealed and the mixture was stirred at 60 °C for 4 h under an argon atmosphere. After cooling to room temperature, the mixture was filtered through a Celite^{®} pad washing with EtOAc several times. The filtrate and washings were combined and the solvent was evaporated to dryness. Purification of the residue by flash chromatography (hexanes/diethyl ether) gave the title compound an oil (43%).

### b) 1-Bromo-2-chloro-4-heptylbenzene

To a solution of 1-Bromo-2-chloro-4-(hept-1-yn-1-yl)benzene in ethanol was added trimethylamine and platinum (IV) oxide and the mixture was stirred under a hydrogen atmosphere for 4 h. The reaction mixture was then filtered through a Celite^{®} pad washing with methanol several times. The filtrate and washings were combined and the solvent was evaporated to dryness. Purification of the residue by flash chromatography (hexanes/diethyl ether) yielded the title compound as an oil (87%).
¹H NMR δ (400 MHz, CDCl₃): 0.84-0.91 (m, 3H), 1.24-1.33 (m, 8H), 1.55-1.61 (m, 2H), 2.49-2.57 (m, 2H), 6.92 (dd, J=8.2, 2.1 Hz, 1H), 7.26 (d, J=2.0 Hz, 1H), 7.48 (d, J=8.2 Hz, 1H).

### c) 2-(2-Chloro-4-heptylphenyl)-4,4,5,5-tetramethyl-1,3,2-dioxaborolane

A Schlenk tube was charged with 1-Bromo-2-chloro-4-heptylbenzene, 4,4,4',4',5,5,5',5'-octamethyl-2,2'-bi(1,3,2-dioxaborolane), potassium acetate and dioxane. The Schlenk tube was subjected to three cycles of evacuation-backfilling with argon and bis(triphenylphosphine)palladium(II) dichloride was added. After three further cycles of evacuation-backfilling with argon, the Schlenk tube was sealed and the mixture was stirred at 90 °C overnight under an argon atmosphere. After cooling to room temperature, the mixture was filtered through a Celite^{®} pad washing with EtOAc several times. The filtrate and washings were combined and the solvents were removed *in vacuo.* Purification of the residue by flash chromatography (hexanes/diethyl ether) gave the title compound as a colourless oil (34%).
¹H NMR δ (400 MHz, CDCl₃): 0.84-0.90 (m, 3H), 1.24-1.32 (m, 8H), 1.36 (s, 12H), 1.56-1.62 (m, 2H), 2.52-2.60 (m, 2H), 7.04 (dd, J=7.6, 1.5 Hz, 1H), 7.17 (d, J=1.2 Hz, 1H), 7.60 (d, J=7.6 Hz, 1H).

### d) Ethyl 4-(2-chloro-4-heptylphenyl)-3-fluoro-1H-pyrrole-2-carboxylate

A Schlenk flask was charged with ethyl 3-fluoro-4-iodo-1H-pyrrole-2-carboxylate, 2-(2-chloro-4-heptylphenyl)-4,4,5,5-tetramethyl-1,3,2-dioxaborolane, dioxane and 2M aqueous cesium carbonate solution. The resulting mixture was subjected to three cycles of evacuation-backfilling with argon and [1,1'-bis(diphenylphosphino)ferrocene]dichloropalladium (II) complex with methylene chloride was then added. After three further cycles of evacuation-backfilling with argon, the Schlenk tube was sealed and the mixture was stirred at 100 °C for 18 h under an argon atmosphere. After cooling to room temperature, the reaction mixture was filtered through a Celite^{®} pad, washing with EtOAc several times. The filtrate and washings were combined, dried over magnesium sulfate and the solvents were removed *in vacuo.* Purification of the residue by flash chromatography (hexanes/diethyl ether) gave the title compound a solid (41%).
MS (m/z): 364 [M-1]⁻.
¹H NMR δ (400 MHz, CDCl₃): 0.85-0.91 (m, 3H), 1.25-1.35 (m, 8H), 1.39 (t, J=7.1 Hz, 3H), 1.59-1.67 (m, 2H), 2.55-2.63 (m, 2H), 4.38 (q, J=7.1 Hz, 2H), 7.04-7.07 (m, 1H), 7.10 (dd, J=7.9, 1.8 Hz, 1H), 7.28 (d, J=1.7 Hz, 1H), 7.35 (dd, J=7.9, 1.2 Hz, 1H), 8.75 (s, 1H).

### Example 1 - 4-(2-Chloro-4-heptylphenyl)-3-fluoro-1H-pyrrole-2-carboxylic acid

To a suspension of ethyl 4-(2-chloro-4-heptylphenyl)-3-fluoro-1H-pyrrole-2-carboxylate in ethanol and water was added lithium hydroxide monohydrate and the mixture was stirred at 70 °C for 4 h. The reaction mixture was cooled to room temperature, the organic solvent was removed *in vacuo,* water was added and the pH of the solution was adjusted to 2-3 by addition of 2N hydrochloric acid solution. The precipitate was filtered, rinsed with water and dried to yield the title compound as an off-white solid (71%).
MS (m/z): 336 [M-1]⁻.
¹H NMR δ (400 MHz, DMSO-*d*₆): 0.82-0.88 (m, 3H), 1.22-1.33 (m, 8H), 1.51-1.63 (m, 2H), 2.55-2.62 (m, 2H), 7.02-7.06 (m, 1H), 7.19 (dd, J=7.9, 1.6 Hz, 1H), 7.31-7.37 (m, 2H), 11.84 (s, 1H), 12.63 (s, 1H).

### EXAMPLE 2 - 4-(2-Ethyl-4-heptylphenyl)-3-fluoro-1H-pyrrole-2-carboxylic acid

### Intermediate C - Ethyl 4-bromo-3-fluoro-1H-pyrrole-2-carboxylate and ethyl 5-bromo-3-fluoro-1H-pyrrole-2-carboxylate

To a solution of ethyl 3-fluoro-1H-pyrrole-2-carboxylate (4.00 g, 25.45 mmol) in DCM (80 mL) was added *N*-bromosuccinimide (4.53 g, 25.45 mmol) and the resulting mixture was stirred in the absence of light for 1h. The reaction mixture was diluted with DCM and washed with water (2 x 40 mL) and brine. The organic layer was dried over magnesium sulfate, filtered and the solvent was evaporated to dryness. The resulting crude was purified by flash chromatography (hexanes/diethyl ether) followed by reverse phase chromatography (water/methanol) to give ethyl 4-bromo-3-fluoro-1H-pyrrole-2-carboxylate (1.60 g, 19%) as a white solid:
MS (m/z): 236, 234 [M, M-2]⁻.
¹H NMR δ (400 MHz, CDCl₃): 1.37 (t, J=7.1 Hz, 3H), 4.36 (q, J=7.1 Hz, 2H), 6.80 (t, J=3.8 Hz, 1H), 8.91 (s, 1H)
and ethyl 5-bromo-3-fluoro-1H-pyrrole-2-carboxylate (3.10 g, 46%) as a white solid:
MS (m/z): 236, 234 [M, M-2]⁻.
   ¹H NMR δ (400 MHz, CDCl₃): 1.36 (t, J=7.1 Hz, 2H), 4.34 (q, J=7.1 Hz, 1H), 6.03 (dd, J=3.2, 0.8 Hz, 1H), 8.81 (s, 1H).

### Intermediate D - Ethyl 4-(2-ethyl-4-heptylphenyl)-3-fluoro-1H-pyrrole-2-carboxylate

### a) 2-Ethyl-4-iodoaniline

To a solution of 2-ethylaniline (5.00 g, 41.37 mmol) in methanol (35 mL) was added a solution of sodium bicarbonate (6.00 g, 71.42 mmol) in water (35 mL). After cooling to 0 °C, diiodine (11.51 g, 45.70 mmol) was added portion wise and the reaction mixture was stirred at room temperature for 2 h. The reaction mixture was then partitioned between DCM and water and phases were separated. The organic layer was washed with saturated aqueous sodium hydrogen sulfite solution, 4% aqueous sodium hydrogen carbonate solution and brine, dried over magnesium sulfate, filtered and the solvents were evaporated to dryness. Purification of the residue by flash chromatography (hexanes/diethyl ether) gave the title compound (8.00 g, 78%) as a yellow oil.
MS (m/z): 248 [M+1]⁺.
¹H NMR δ (400 MHz, CDCl₃): 1.23 (t, J=7.5 Hz, 3H), 2.45 (q, J=7.5 Hz, 2H), 3.63 (s, 2H), 6.45 (d, J=8.3 Hz, 1H), 7.29 (dd, J=8.3, 2.1 Hz, 1H), 7.33 (d, J=2.1 Hz, 1H).

### b) 1-Bromo-2-ethyl-4-iodobenzene

To a solution of 2-ethyl-4-iodoaniline (4.00 g, 16.19 mmol) in ACN (40 mL) were added tetrabutylammonium bromide (10.44 g, 32.26 mmol), 4-methyl benzenesulfonic acid monohydrate (3.06 g, 16.09 mmol), tert-butyl nitrite (2.3 mL, 19.36 mmol) and copper (II) bromide (3.6 mg, 0.016 mmol). The resulting mixture was stirred at room temperature for 5 h, then poured into water and extracted with EtOAc (x3). The combined organic layers were washed with brine, dried over magnesium sulfate, filtered and the solvents were evaporated to dryness. Purification of the residue by flash chromatography (hexanes/diethyl ether) gave the title compound (3.92 g, 78%) as an orange oil.
¹H NMR δ (400 MHz, CDCl₃): 1.21 (t, J=7.5 Hz, 3H), 2.70 (q, J=7.5 Hz, 2H), 7.24 (d, J=8.3 Hz, 1H), 7.35 (dd, J=8.3, 2.2 Hz, 1H), 7.55 (d, J=2.2 Hz, 1H).

### c) 1-Bromo-2-ethyl-4-(hept-1-yn-1-yl)benzene

A Schlenk tube was charged with 1-bromo-2-ethyl-4-iodobenzene, hept-1-yne, DCM and TEA. The Schlenk tube was subjected to three cycles of evacuation-backfilling with argon and then tetrakis(triphenylphosphine)palladium(0) and copper (I) iodide were added. After three further cycles of evacuation-backfilling with argon, the Schlenk tube was sealed and the mixture was stirred at 60 °C for 4 h under an argon atmosphere. After cooling to room temperature, the mixture was filtered through a Celite^{®} pad washing with EtOAc several times. The filtrate and washings were combined and the solvent was evaporated to dryness. Purification of the residue by flash chromatography (hexanes/diethyl ether) gave the title compound as a yellow oil (65%).
¹H NMR δ (400 MHz, CDCl₃): 0.92 (t, J=7.1 Hz, 4H), 1.21 (t, J=7.5 Hz, 3H), 1.32-1.47 (m, 4H), 1.56-1.64 (m, 2H), 2.38 (t, J=7.1 Hz, 2H), 2.71 (q, J=7.5 Hz, 2H), 7.06 (dd, J=8.2, 2.1 Hz, 1H), 7.26 (d, J=2.0 Hz, 1H), 7.42 (d, J=8.2 Hz, 1H).

### d) 1-Bromo-2-ethyl-4-heptylbenzene

To a solution of 1-bromo-2-ethyl-4-(hept-1-yn-1-yl)benzene in ethanol (20 mL) was added trimethylamine and platinum (IV) oxide and the mixture was stirred under a hydrogen atmosphere for 4 h. The reaction mixture was then filtered through a Celite^{®} pad washing with methanol several times.

The filtrate and washings were combined and the solvent was evaporated to dryness. Purification of the residue by flash chromatography (hexanes/diethyl ether) yielded the title compound as a colourless oil (74%).
¹H NMR δ (400 MHz, CDCl₃): 0.85-0.90 (m, 3H), 1.19-1.23 (m, 3H), 1.27-1.33 (m, 8H), 1.55-1.62 (m, 2H), 2.50-2.56 (m, 2H), 2.72 (q, J=7.5 Hz, 2H), 6.86 (dd, J=8.1, 2.2 Hz, 1H), 7.03 (d, J=2.1 Hz, 1H), 7.40 (d, J=8.1 Hz, 1H).

### e) 2-(2-Ethyl-4-heptylphenyl)-4,4,5,5-tetramethyl-1,3,2-dioxaborolane

A Schlenk tube was charged with 1-bromo-2-ethyl-4-heptylbenzene, 4,4,4',4',5,5,5',5'-octamethyl-2,2'-bi(1,3,2-dioxaborolane), potassium acetate and dioxane. The Schlenk tube was subjected to three cycles of evacuation-backfilling with argon and bis(triphenylphosphine)palladium(II) dichloride was added. After three further cycles of evacuation-backfilling with argon, the Schlenk tube was sealed and the mixture was stirred at 90 °C overnight under an argon atmosphere. After cooling to room temperature, the mixture was filtered through a Celite^{®} pad washing with EtOAc several times. The filtrate and washings were combined and the solvents were removed *in vacuo.* Purification of the residue by flash chromatography (hexanes/diethyl ether) gave the title compound as a yellow solid (65%).
¹H NMR δ (400 MHz, CDCl₃): 0.84-0.91 (m, 3H), 1.19 (t, J=7.5 Hz, 3H), 1.25-1.31 (m, 8H), 1.33 (s, 12H), 1.57-1.64 (m, 2H), 2.54-2.60 (m, 2H), 2.89 (q, J=7.5 Hz, 2H), 6.98-7.01 (m, 2H), 7.69 (d, J=8.1 Hz, 1H).

### f) Ethyl 4-(2-ethyl-4-heptylphenyl)-3-fluoro-1H-pyrrole-2-carboxylate

A Schlenk flask was charged with ethyl 4-bromo-3-fluoro-1H-pyrrole-2-carboxylate, 2-(2-ethyl-4-heptylphenyl)-4,4,5,5-tetramethyl-1,3,2-dioxaborolane, dioxane and 2M aqueous cesium carbonate solution. The resulting mixture was subjected to three cycles of evacuation-backfilling with argon and [1,1'-bis(diphenylphosphino)ferrocene]dichloropalladium (II) complex with methylene chloride was then added. After three further cycles of evacuation-backfilling with argon, the Schlenk tube was sealed and the mixture was stirred at 100 °C for 18 h under an argon atmosphere. After cooling to room temperature, the reaction mixture was filtered through a Celite^{®} pad, washing with EtOAc several times. The filtrate and washings were combined, dried over magnesium sulfate and the solvents were removed *in vacuo.* Purification of the residue by flash chromatography (hexanes/diethyl ether) gave the title compound as a colourless oil (53%).
MS (m/z): 358 [M-1]⁻.
¹H NMR δ (400 MHz, CDCl₃): 0.87-0.91 (m, 3H), 1.15 (t, J=7.5 Hz, 3H), 1.25-1.36 (m, 8H), 1.38 (t, J=7.1 Hz, 3H), 1.59-1.69 (m, 2H), 2.57-2.69 (m, 4H), 4.37 (q, J=7.1 Hz, 2H), 6.75 (dd, J=4.4, 3.7 Hz, 1H), 7.03 (dd, J=7.7, 1.8 Hz, 1H), 7.11-7.16 (m, 2H), 8.67 (s, 1H).

### Example 2 - 4-(2-Ethyl-4-heptylphenyl)-3-fluoro-1H-pyrrole-2-carboxylic acid

To a suspension of ethyl 4-(2-ethyl-4-heptylphenyl)-3-fluoro-1H-pyrrole-2-carboxylate in ethanol and water was added lithium hydroxide monohydrate and the mixture was stirred at 70 °C for 4 h. The reaction mixture was cooled to room temperature, the organic solvent was removed *in vacuo,* water was added and the pH of the solution was adjusted to 2-3 by addition of 2N hydrochloric acid solution. Obtained as a white solid (41%) following purification of the crude product by reverse phase chromatography (water/methanol).
MS (m/z): 330 [M-1]⁻.
¹H NMR δ (400 MHz, DMSO-*d*₆): 0.83-0.89 (m, 3H), 1.06 (t, J=7.5 Hz, 3H), 1.22-1.34 (m, 9H), 1.52-1.62 (m, 2H), 2.52-2.59 (m, 4H), 6.86-6.89 (m, 1H), 7.01 (dd, J=7.8, 1.5 Hz, 1H), 7.06-7.12 (m, 2H), 11.71 (s, 1H), 12.55 (s, 1H).

### EXAMPLE 3 - 3-Fluoro-4-tridecyl-1H-pyrrole-2-carboxylic acid

### Intermediate E - Ethyl 3-fluoro-4-tridecanoyl-1H-pyrrole-2-carboxylate

### a) Tridecanoyl chloride

To a cooled (0 °C) solution of tridecanoic acid in DCM was added dropwise oxalyl chloride and DMF and the mixture was stirred at room temperature for 4 h. The solvent was removed under reduced pressure to yield the title compound as an oil (100%) which was used in the next synthetic step without further purification.

### b) Ethyl 3-fluoro-4-tridecanoyl-1H-pyrrole-2-carboxylate

To a solution of tridecanoyl chloride in DCM under argon atmosphere at 0 °C was added portionwise aluminium(III) chloride followed by a solution of ethyl 3-fluoro-1H-pyrrole-2-carboxylate in DCM and the mixture was stirred at room temperature for three days. After cooling to 0 °C, 1N hydrochloric acid solution was added dropwise and the reaction mixture was partitioned between EtOAc and water. The organic phase was separated and the aqueous phase was extracted with EtOAc (x2). The combined organic extracts were washed with brine, dried over magnesium sulfate, filtered and the solvent was evaporated to dryness. The residue was purified by flash chromatography (hexanes/diethyl ether) to yield the title compound as a white solid (68%).
MS (m/z): 354 [M+1]⁺
¹H-NMR δ (400 MHz, CDCl₃): 0.96-0.72 (m, 3H), 1.26 (s, 18H), 1.39 (t, J=7 Hz, 3H), 1.68 (p, J=8 Hz, 2H), 2.77 (t, J=8 Hz, 2H), 4.38 (q, J=7 Hz, 2H), 7.35 (s, 1H), 9.03 (brs, 1H).

### c) Ethyl 3-fluoro-4-tridecyl-1H-pyrrole-2-carboxylate

To a solution of ethyl **3-fluoro-4-tridecanoyl-1H-pyrrole-2-carboxylate** in TFA was added dropwise triethylsilane and the mixture was stirred at room temperature for 3 h. TFA was evaporated and the residue was partitioned between DCM and saturated aqueous sodium hydrogen carbonate solution. The organic layer was separated, washed with saturated aqueous sodium hydrogen carbonate solution (x2) and brine, dried over magnesium sulfate, filtered and the solvent was evaporated to dryness. The residue was purified by flash chromatography (hexanes/diethyl ether) to yield the title compound as a white solid (70%).
MS (m/z): 340 [M+1]⁺
¹ H-NMR 5 (400 MHz, CDCl₃): 0.93-0.82 (m, 3H), 1.26 (s, 20H), 1.36 (t, J=7 Hz, 3H), 1.55 (dd, J=13 and 6 Hz, 2H), 2.46-2.34 (m, 2H), 4.33 (q, J=7 Hz, 2H), 6.66-6.41 (m, 1 H), 8.41 (brs, 1 H).

### Example 3 - 3-Fluoro-4-tridecyl-1H-pyrrole-2-carboxylic acid

To a solution of ethyl 3-fluoro-4-tridecyl-1H-pyrrole-2-carboxylate (2379 mg, 7.01 mmol) in ethanol (60 mL) was added sodium hydroxide (981 mg, 24.53 mmol) and the mixture was heated to reflux overnight. The volatiles were removed under reduced pressure, water was added and pH lowered to 2 by addition of 1N hydrochloric acid solution. The solid formed was filtered, washed with water (x3) and dried to yield the title compound (2097 mg, 95%) as a white solid.
MS (m/z): 312 [M+1]⁺
¹H-NMR δ (400 MHz, DMSO-*d*₆): 0.92-0.75 (m, 3H), 1.23 (m, 20H), 1.52-1.41 (m, 2H), 2.32 (t, J=7 Hz, 2H), 6.75-6.52 (m, 1H), 11.34-11.16 (m, 1H).

### EXAMPLE 4 - 3-Fluoro-4-((1r,4s)-4-octylcyclohexyl)-1H-pyrrole-2-carboxylic acid

### Intermediate F - Ethyl 3-fluoro-4-((1r,4s)-4-octylcyclohexyl)-1H-pyrrole-2-carboxylate and ethyl 3-fluoro-4-((1s,4r)-4-octylcyclohexyl)-1H-pyrrole-2-carboxylate

### a) 4-Octylcyclohex-1-en-1-yl trifluoromethanesulfonate

To a cooled (-78 °C) solution of 4-octylcyclohexan-1-one (1.00 g, 4.75 mmol) in THF (12 mL) was added lithium bis(trimethylsilyl)amide (1M solution in THF, 5.3 mL, 5.30 mmol) under argon atmosphere and the resulting solution was stirred at -78 ºC for 1 h. A solution of 1,1,1-trifluoro-*N*-phenyl-*N-*((trifluoromethyl)sulfonyl)methanesulfonamide (1.87 g, 5.23 mmol) in THF (8 mL) was then slowly added. The cooling bath was removed and the reaction mixture was stirred for further 3 h. Water and diethyl ether were added, the organic layer was separated and the aqueous layer was extracted with diethyl ether (x2). The combined organic layers were washed with saturated aqueous sodium hydrogen carbonate solution and brine, dried over magnesium sulfate, filtered and the solvents were evaporated to yield the title compound as an orange oil (1.63 g, 98%).
¹H NMR δ (400 MHz, CDCl₃): 0.88 (t, J=6.8 Hz, 3H), 1.20-1.33 (m, 12H), 1.38-1.46 (m, 1H), 1.50-1.60 (m, 2H), 1.75-1.91 (m, 2H), 2.20-2.42 (m, 4H), 5.71 (dd, J=4.9, 2.3 Hz, 1H).

### b) 4,4,5,5-Tetramethyl-2-(4-octylcyclohex-1-en-1-yl)-1,3,2-dioxaborolane

A Schlenk tube was charged with 4-octylcyclohex-1-en-1-yl trifluoromethanesulfonate, 4,4,4',4',5,5,5',5'-octamethyl-2,2'-bi(1,3,2-dioxaborolane), potassium acetate and dioxane. The Schlenk tube was subjected to three cycles of evacuation-backfilling with argon and bis(triphenylphosphine)palladium(II) dichloride was added. After three further cycles of evacuation-backfilling with argon, the Schlenk tube was sealed and the mixture was stirred at 90 °C overnight under an argon atmosphere. After cooling to room temperature, the mixture was filtered through a Celite^{®} pad washing with EtOAc several times. The filtrate and washings were combined and the solvents were removed *in vacuo.* Purification of the residue by flash chromatography (hexanes/diethyl ether) gave the title compound as a colourless oil (40%).

### c) Ethyl 3-fluoro-4-(4-octylcyclohex-1-en-1-yl)-1H-pyrrole-2-carboxylate

A Schlenk flask was charged with ethyl 3-fluoro-4-iodo-1H-pyrrole-2-carboxylate (Intermediate A), 4,4,5,5-tetramethyl-2-(4-octylcyclohex-1-en-1-yl)-1,3,2-dioxaborolane, dioxane and 2M aqueous cesium carbonate solution. The resulting mixture was subjected to three cycles of evacuation-backfilling with argon and [1,1'-bis(diphenylphosphino)ferrocene]dichloropalladium (II) complex with methylene chloride was then added. After three further cycles of evacuation-backfilling with argon, the Schlenk tube was sealed and the mixture was stirred at 100 °C for 18 h under an argon atmosphere. After cooling to room temperature, the reaction mixture was filtered through a Celite^{®} pad, washing with EtOAc several times. The filtrate and washings were combined, dried over magnesium sulfate and the solvents were removed *in vacuo.* Purification of the residue by flash chromatography (hexanes/diethyl ether) gave the title compound as a light yellow solid (56%).
MS (m/z): 348 [M-1]⁻.
¹H NMR δ (400 MHz, CDCl₃): 0.86-0.91 (m, 3H), 1.25-1.33 (m, 16H), 1.36 (t, J=7.1 Hz, 3H), 1.73-1.89 (m, 3H), 2.20-2.33 (m, 3H), 4.34 (q, J=7.1 Hz, 2H), 6.12-6.15 (m, 1H), 6.66-6.70 (m, 1H), 8.45 (s, 1H).

### d) Ethyl 3-fluoro-4-((1r,4s)-4-octylcyclohexyl)-1H-pyrrole-2-carboxylate and ethyl 3-fluoro-4-((1s,4r)-4-octylcyclohexyl)-1H-pyrrole-2-carboxylate

To a solution of ethyl 3-fluoro-4-(4-octylcyclohex-1-en-1-yl)-1H-pyrrole-2-carboxylate (230 mg, 0.66 mmol) in a mixture of methanol (6 mL) and ethanol (2 mL) was added 10% palladium on carbon (23 mg, 0.21 mmol) and the resulting suspension was stirred under a hydrogen atmosphere overnight. The catalyst was filtered through a Celite^{®} pad, washing with methanol several times. The filtrate and washings were combined and the solvents were evaporated to give the title compounds (231 mg, 100%) as a white solid.
MS (m/z): 352 [M+l]⁺.

### Example 4 - 3-Fluoro-4-((1r,4s)-4-octylcyclohexyl)-1H-pyrrole-2-carboxylic acid

To a suspension of ethyl 3-fluoro-4-((1*r*,4*s*)-4-octylcyclohexyl)-1H-pyrrole-2-carboxylate and ethyl 3-fluoro-4-((1*s*,4*r*)-4-octylcyclohexyl) -1H-pyrrole-2-carboxylate in ethanol and water was added lithium hydroxide monohydrate and the mixture was stirred at 70 ºC for 4 h. The reaction mixture was cooled to room temperature, the organic solvent was removed *in vacuo,* water was added and the pH of the solution was adjusted to 2-3 by addition of 2N hydrochloric acid solution. The precipitate was filtered, rinsed with water and dried to yield the title compound as a white solid (42%). Purification of the crude product by reverse phase chromatography (water/methanol) provided pure samples of both isomers:
First eluting isomer - Example 4
   MS (m/z): 322 [M-1]⁻.
   ¹H NMR δ (400 MHz, DMSO-d₆): 0.82-0.88 (m, 3H), 1.21-1.31 (m, 14H), 1.35-1.44 (m, 2H), 1.46-1.68 (m, 7H), 2.55-2.64 (m, 1H), 6.62-6.67 (m, 1H), 11.25 (s, 1H), 12.32 (s, 2H).
Second eluting isomer
   MS (m/z): 322 [M-1]⁻.
   ¹H NMR δ (400 MHz, DMSO-*d*₆): 0.83-0.88 (m, 3H), 0.90-1.04 (m, 2H), 1.14-1.34 (m, 17H), 1.72-1.87 (m, 4H), 2.31-2.37 (m, 1H), 6.59-6.62 (m, 1H), 11.23 (s, 1H), 12.31 (s, 1H).

### Pharmacological activity of the pyrrole compounds

### In vitro assay of inhibition of Lipid Synthesis

To evaluate the inhibition of lipid synthesis, the immortalized human sebocyte cell line, SZ95 (stablished by Zouboulis, C.C. et al J Invest Dermatol 1999; 113: 1011-20), was treated with arachidonic acid (AA) in presence or absence of compound. Lipids were detected by using a lipid sensing fluorophore.

10k cells were plated in 384 well microtiter plates and incubated at 37 °C and 5% CO₂ in DMEM/F12 supplemented with 10% FBS, 1.25ng/ml of rhEGF and GA-1000, using the MicroClime Lids from Labcyte to reduce the edge effect, before of compound and stimulus addition.

After 24h, compounds were dissolved in DMSO 100%. Then the stocks were serial diluted 1/3 in DMSO 100%, and this battery of solutions were diluted 1/10 in culture medium. Later, compounds dissolved in culture media were added over cells, diluting the solutions prepared 1/40 in the final volume of the assay. Then, cells and compounds were preincubated for 30 min at 37ºC and 5% CO2. After this prior incubation, the lipid synthesis was induced by 75µM of AA final solution, preparing a solution 10x in culture media containing a 1.25% of DMSO. Finally, SZ95 treated were incubated for 48h at 37°C and 5% CO₂.

Neutral lipids were measured using AdipoRedTM, purchased from Lonza. To do that, cells were washed with PBS and incubated with a solution of AdipoRedTM (final dilution 1/80 in PBS) for 30 min at room temperature. After the staining process, the fluorescence intensity (FI) was quantified using a fluorescence plate reader (excitation 485 nm; emission 535).

Activity of compounds was calculated as % of inhibition considering the maximal fluorescence for AA-stimulated cells and the minimum fluorescence for unstimulated cells as controls.

Some of the acronyms used above have the following meaning:
AA: Arachidonic Acid
DMSO: dimethylsulfoxide
DMEM/F12: Dulbecco's Modified Eagle's Medium/F12
FBS: Fetal Bovine Serum
rhEGF: recombinant human Epidermal Growth Factor
GA-1000: Gentamicin/Amphotericin
PBS: Phosphate-buffered saline
FI: Fluorescence intensity

The IC₅₀ values for Examples 1, 2 and 4 were all less than 200 nM. The IC₅₀ values for Example 3 was less than 50 nM. The pyrrole compounds are, therefore, potent inhibitors of lipid synthesis.

Preferred pyrrole derivatives of the invention possess an IC₅₀ value for the inhibition of lipid synthesis (determined as defined above) of less than 1 µM (1000 nM), preferably of less than 0.20 µM (200 nM).

### General preparation of the topical gel composition

All gel batches were manufactured at a lab scale from 10g to 50kg.

In the case of microsuspensions, the topical gel composition of the invention may be obtained using a dispersing device (i.e. Ultra-turrax^{®}) or an homogenizer mixer (i.e. Becomix).

The microsuspensions described herein were prepared according to the following process comprising the following steps:
a) a first step of preparing a solution of the preservatives;
b) a second step of dissolving the surfactant into water in a dispersing device or an homogenizer mixer;
c) a third step of adding the solution of step a) into the mixture of step b);
d) a fourth step of adding, with homogenization, the pyrrole compound of Formula (I) into the solution of step c); and
e) a fifth step of incorporating, with homogenization, the gelling agent into the suspension of step d).

In the case of nanosuspensions, the topical gel composition of the invention may be obtained using high pressure homogenization techniques. Suitable equipment to obtain the topical gel composition of the invention include the LV1 Microfluidizer^{®} High Shear Fluid Processor and PANDA-GEA High Pressure Homogenizer.

The nanosuspensions described herein were prepared according to the following process comprising the following steps:
a) a first step of preparing a solution of the preservatives;
b) a second step of dissolving the surfactant agent in water;
c) a third step of adding the solution of step a) into the solution of step b);
d) a fourth step of adding the pyrrole compound of Formula (I) into the solution of step c);
e) a fifth step of dispersing the suspension of step d) with an Ultra-turrax^{®} disperser;
f) a sixth step of homogenizing the above suspension with a PANDA-GEA High Pressure homogenizer until the appropriate particle size is obtained (i.e. 300 - 800 nm); and
g) a final step of adding the gelling agent into the suspension of step f).

In case that the pyrrole compound is dissolved within the topical gel composition, the resulting emulsion described herein was prepared according to the following process comprising the following steps:
a) a first step of preparing a solution of the hydrophilic ingredients in water;
b) a second step of preparing a solution of the lipophilic ingredients in the lipophilic solvent;
c) a third step of adding, dropwise, the solution of step b) into the solution of step a);
d) a fourth step of dispersing the resulting emulsion with an Ultra-turrax^{®} disperser; and
e) a final step of adding the gelling agent.

### Examples

Topical gel compositions of the invention are set out in the examples below. The compositions all form gels. In the case of Examples 1 to 3 the pyrrole compound of Formula (I) is dissolved within the topical gel composition, whereas in the case of Examples 4-10 the formulations are suspension gels. In the case of suspensions gels unless stated otherwise, the pyrrole compound is micronized, thus forming a microsuspension gel. The invention is illustrated by means of the following examples which must not be considered as limiting.

### Example 1

| **Trade name** | **INCI name** | **%** |
|---|---|---|
| 4-(2-Chloro-4-heptylphenyl)-3-fluoro-1H-pyrrole-2-carboxylic acid | 4-(2-Chloro-4-heptylphenyl)-3-fluoro-1H-pyrrole-2-carboxylic acid | 1 |
| Diisopropyl adipate | Diisopropyl adipate | 7 |
| Lauroglycol^{™} FCC | Propylene glycol | 3 |
| | monolaurate | |
| Kollicream^{®} OD | Octyldodecanol | 10 |
| Sepineo ^{™} P600 | Acrylamide | 3 |
| | Sodium acryloyldimethyl | |
| | taurate copolymer | |
| | Isohexadecane | |
| | Polysorbate 80 | |
| Tween^{™} 80 | Polysorbate 80 | 1 |
| Nipagin | Methylparabene | 0.2 |
| Nipasol | Propylparabene | 0.02 |
| Phenoxyethanol | Phenoxyethanol | 1.0 |
| Water | Water | 58.78 |

### Example 2

| **Trade name** | **INCI name** | **%** |
|---|---|---|
| 4-(2-Chloro-4-heptylphenyl)-3-fluoro-1H-pyrrole-2-carboxylic acid | 4-(2-Chloro-4-heptylphenyl)-3-fluoro-1H-pyrrole-2-carboxylic acid | 1 |
| Diisopropyl adipate | Diisopropyl adipate | 7 |
| Lauroglycol^{™} FCC | Propylene glycol | 3 |
| | monolaurate | |
| Sepineo ^{™} P600 | Acrylamide | 2 |
| | Sodium acryloyldimethyl | |
| | taurate copolymer | |
| | Isohexadecane | |
| | Polysorbate 80 | |
| Liquid vaseline | Petrolatum | 20 |
| water | water | 67 |

### Example 3

| **Trade name** | **INCI name** | **%** |
|---|---|---|
| 4-(2 -Ethyl-4-heptyl phenyl)- 3-fluoro-1H-pyrrole-2-carboxylic acid | 4-(2- Ethyl-4-heptyl ph enyl)- 3-fluoro-1H-pyrrole-2-carboxylic acid | 1 |
| Diisopropyl adipate | Diisopropyl adipate | 10 |
| Kollisolv^{®} MCT 70 | Capric Triglyceride | 15 |
| Sepineo^{™} DERM | Hydroxyethyl acrylate, sodium acryloyldimethyl taurate copolymer | 2 |
| | Polysorbate 80 | |
| Lauroglycol^{™} FCC | Propylene glycol monolaurate | 5 |
| Labrasol^{®} | PEG-8 Caprylic/Capric | 2 |
| | Glycerides | 10 |
| Kollicream^{®} OD | Octyldodecanol | 0.5 |
| Benzyl alcohol | Benzyl alcohol | 5 |
| Glycerin | Glycerin | |
| Water | Water | 49.5 |

### Example 4

| **Trade name** | **INCI name** | **%** |
|---|---|---|
| 4-(2-Chloro-4-heptylphenyl)-3-fluoro-1H-pyrrole-2-carboxylic acid | 4-(2-Chloro-4-heptylphenyl)-3-fluoro-1H-pyrrole-2-carboxylic acid | 1 |
| Tween^{™} 80 | Polysorbate 80 | 0.1 |
| Methylcellulose | Methylcellulose | 0.5 |
| Water | Water | 98.4 |

### Example 5

| **Trade name** | **INCI name** | **%** |
|---|---|---|
| 3-Fluoro-4-tridecyl-1H-pyrrole-2-carboxylic acid | 3-Fluoro-4-tridecyl-1 H-pyrrole-2-carboxylic acid | 3 |
| Tween ^{™} 80 | Polysorbate 80 | 2 |
| Sepineo ^{™} P600 | Acrylamide | 3 |
| | Sodium acryloyldimethyl taurate copolymer | |
| | Isohexadecane | |
| | Polysorbate 80 | |
| Phenoxyethanol | Phenoxyethanol | 1 |
| Nipagin | Methylparabene | 0,2 |
| Nipasol | Propylparabene | 0,02 |
| Water | Water | 90,78 |

### Example 6

| **Trade name** | **INCI name** | **%** |
|---|---|---|
| 3-Fluoro-4-tridecyl-1H-pyrrole-2-carboxylic acid | 3-Fluoro-4-tridecyl-1H-pyrrole-2-carboxylic acid | 5 |
| Tween^{™} 80 | Polysorbate 80 | 2 |
| Sepineo ^{™} P600 | Acrylamide | 3 |
| | Sodium acryloyldimethyl taurate copolymer | |
| | Isohexadecane | |
| | Polysorbate 80 | |
| Nipagin | Methylparabene | 0,2 |
| Nipasol | Propylparabene | 0,02 |
| Phenoxyethanol | Phenoxyethanol | 1 |
| Water | Water | 88,78 |

### Examples 7a and 7b

| **Trade name** | **INCI name** | **%(7a)** | **%(7b)** |
|---|---|---|---|
| 3-Fluoro-4-tridecyl-1H-pyrrole-2-carboxylic acid | 3-Fluoro-4-tridecyl-1H-pyrrole-2-carboxylic acid | 1 | 0.01 |
| Tween^{™} 80 | Polysorbate 80 | 0.66 | 0.0066 |
| Sepineo ^{™} P600 | Acrylamide | 3 | 3 |
| | Sodium acryloyldimethyl taurate copolymer | | |
| | Isohexadecane | | |
| | Polysorbate 80 | | |
| Nipagin | Methylparabene | 0.2 | 0.2 |
| Nipasol | Propylparabene | 0.02 | 0.02 |
| Phenoxyethanol | Phenoxyethanol | 1 | 1 |
| Water | Water | 94.12 | 95.76 |

### Example 8

| **Trade name** | **INCI name** | **%** |
|---|---|---|
| 3-Fluoro-4-tridecyl-1H-pyrrole-2-carboxylic acid | 3-Fluoro-4-tridecyl-1 H-pyrrole-2-carboxylic acid | 1 |
| Tween ^{™} 20 | Polysorbate 20 | 2 |
| Xanthan gum | Xanthan gum | 2 |
| Water | Water | 95 |

### Example 9

| **Trade name** | **INCI name** | **%** |
|---|---|---|
| 3-Fluoro-4-tridecyl-1H-pyrrole-2-carboxylic acid | 3-Fluoro-4-tridecyl-1H-pyrrole-2-carboxylic acid | 1 |
| Arlamol^{™} PS11E-LQ | PPG-15 Stearyl Ether | 2 |
| Sepineo ^{™} P600 | Acrylamide | 2 |
| | Sodium acryloyldimethyl taurate copolymer | |
| | Isohexadecane | |
| | Polysorbate 80 | |
| Water | Water | 95 |

### Example 10

| **Trade name** | **INCI name** | **%** |
|---|---|---|
| 3-Fluoro-4-((1r,4s)-4-octylcyclohexyl)-1H-pyrrole-2-carboxylic acid | 3-Fluoro-4-((1r,4s)-4-octylcyclohexyl)-1H-pyrrole-2-carboxylic acid | 1 |
| Tween^{™} 80 | Polysorbate 80 | 0.66 |
| Sepineo^{™} P600 | Acrylamide Sodium acryloyldimethyl taurate copolymer Isohexadecane Polysorbate 80 | 2 |
| Water | Water | 96.34 |

### Examples 11a and 11b

| Trade name | **INCI name** | % (11a) | % (11b) |
|---|---|---|---|
| 3-Fluoro-4-tridecyl-1H-pyrrole-2-carboxylic acid (nanonized) | 3-Fluoro-4-tridecyl-1H-pyrrole-2-carboxylic acid (nanonized) | 3 | 1 |
| Tween ^{™} 80 | Polysorbate 80 | 2 | 2 |
| Sepineo ^{™} P600 | Acrylamide Sodium acryloyldimethyl taurate copolymer Isohexadecane Polysorbate 80 | 2 | 2 |
| Water | Water | 93 | 95 |

### Gel morphology

The morphology of the suspension gels shown in the above examples was determined by microscopy (Nikon Eclipse 50i). The optical microscopic observation showed the presence of nanoparticles or microparticles uniformly distributed through the gel. This observation was done for samples at initial time t=0, and in some cases were also observed after preliminary stability (see Figure 1). As it can be observed, the physical characteristics of the gels shows good stability during the studied period, with homogeneous particle distribution. Neither agglomeration nor recrystallization of particles of the active agent was observed.

### Particle size

The particle size of gels comprising a micronized pyrrole compound of Formula (I) is measured using a particle size analyzer (Helos, from Sympatec Gmbh). The particle size should preferably be from 1 µm to 20 µm. More preferably from 1 µm to 10 µm, even more preferably from 4 µm to 7 µm.

The particle size of gels comprising a nanonized pyrrole compound of Formula (I) is measured using a ZetasizerNano (Malvern). As an example, the particle size of a batch of formulations in Examples 11a and 11b are shown in Table 1 below:

**Table 1**

| **Example** | **Z-Average** | **Pdl** |
|---|---|---|
| 11a | 319 nm | 0.260 |
| 11b | 305 nm | 0.138 |

### Texture analysis

Textural analysis of some examples shown above has also been carried out, using a TA.XT 2 Stable Micro System's texture analyzer, to assess the hardness and adhesiveness of the gel composition. The results are provided in Tables 2 below:

**Table 2**

| **Example** | **Hardness (g)** | **Adhesiveness (g.sec)** |
|---|---|---|
| 5 | 10.101 | -34.002 |
| 6 | 10.973 | -37.927 |
| 7a | 9.883 | -33.792 |

The texture analysis demonstrates that the gel compositions are suitable for use as topical formulations for application to the skin, showing a better sensory profile than well-established commercial products.

## Claims

1. A topical gel composition comprising:
a) a pyrrole compound, which pyrrole compound is a compound of Formula (I) or a pharmaceutically acceptable salt thereof: 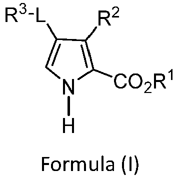 wherein:
• R¹ is selected from the group consisting of a hydrogen atom and a linear or branched C₁₋₄ alkyl group;
• R² represents a hydrogen atom or a halogen atom;
• L is selected from the group consisting of a direct bond, a C₅₋₆ cycloalkyl group and a phenyl ring, wherein the cycloalkyl group and the phenyl ring are unsubstituted or substituted by one or more substituents selected from a halogen atom and a linear or branched C₁₋₄alkyl group; and
• R³ represents a linear or branched C₇₋₁₆ alkyl group;
(b) water, and
(c) a gelling agent.

2. A topical gel composition according to claim 1, wherein R¹ represents a hydrogen atom.

3. A topical gel composition according to any one of the preceding claims, wherein R² represents a fluorine atom or a chlorine atom, preferably a fluorine atom.

4. A topical gel composition according to any one of the preceding claims wherein L represents a direct bond or a phenyl ring, wherein the phenyl ring is unsubstituted or substituted by one or two substituents selected from a halogen atom and a linear C₁₋₂alkyl group.

5. A topical gel composition according to any one of the preceding claims, wherein R³ represent a linear or branched C₁₂₋₁₃ alkyl group.

6. A topical gel composition according to any one of the preceding claims, wherein the pyrrole compound is 3-fluoro-4-tridecyl-1H-pyrrole-2-carboxylic acid or a pharmaceutically acceptable salt thereof.

7. A topical gel composition according to any one of the preceding claims further comprising a surfactant.

8. A topical gel composition according to any one of the preceding claims further comprising a preservative system comprising one or more preservatives.

9. A topical gel composition according to any one of the preceding claims optionally comprising a moisturizer.

10. A topical gel composition according to any one of the preceding claims, which contains a suspension of particles, which particles comprise the pyrrole compound.

11. A topical gel composition according to claim 10, in which the particles forming said suspension have a particle size of 50 nm to 100 µm, for example from 100 nm to 50 µm, preferably from 1 µm to 20 µm. More preferably from 1 µm to 10 µm, even more preferably from 4 µm to 7 µm.

12. A topical gel composition according to any one of the preceding claims wherein the pyrrole compound is present in an amount of from 0.005% to 10% by weight of the total composition, preferably in an amount of from 0.5% to 7.5 % by weight of the total composition.

13. A topical gel composition according to any one of the preceding claims wherein the water is present in an amount of at least 45% by weight of the total composition, preferably at least 55% by weight of the total composition, more preferably at least 80% by weight of the total composition, even more preferably at least 85% by weight of the total composition.

14. A topical gel composition according to any one of the preceding claims wherein the gelling agent is selected from the group consisting of natural gelling agents, polymeric gelling agents and cellulose-based gelling agents, preferably polymeric gelling agents.

15. A topical gel composition according to any one of the preceding claims, wherein the gelling agent is selected from the group consisting of xanthan gum, a Acrylamide/Sodium Acryloyldimethyl Taurate Copolymer/Isohexadecane & Polysorbate 80 mixture (sold under the name Sepineo^{™} P600), Hydroxyethyl Acrylate/Sodium Acryloyldimethyl Taurate Copolymer (sold under the name Sepineo^{™} DERM), Acrylates/C10-30 Alkyl Acrylate Crosspolymer (Pemulen^{™} TR-2), Methylcellulose, Hydroxypropyl Methylcellulose, preferably a Acrylamide/Sodium Acryloyldimethyl Taurate Copolymer/lsohexadecane & Polysorbate 80 mixture (Sepineo^{™} P600).

16. A topical gel composition according to any one of the preceding claims wherein the gelling agent is present in an amount from 0.5 % to 5% by weight of the total composition, preferably from 1% to 4% by weight of the total composition, more preferably from 2.5 to 3.5 % by weight of the total composition.

17. A topical gel composition according to any one of claims 7 to 16, wherein the surfactant is selected from the group consisting of non-ionic surfactants, a cationic surfactant, an anionic surfactant, a zwitterionic surfactant, and a mixture thereof.

18. A topical gel composition according to any one of claims 7 to 17, wherein the surfactant is selected from the group consisting of PEG-8 Caprylic/Capric Glycerides (sold under the name Labrasol^{®}), Propylene glycol monolaurate (sold under the name Lauroglycol^{™} FCC), Polysorbate 20, Polysorbate 80, PPG-15 Stearyl Ether (sold under the name Arlamol^{™} PS11E) or a mixture thereof, preferably Arlamol^{™} PS11E and Polysorbate 80, more preferably Polysorbate 80.

19. A topical gel composition according to any one of claims 7 to 18, wherein the surfactant is present in an amount of from 0 % to 7 % by weight of the total composition, preferably from 0.2 % to 5% by weight of the total composition, more preferably from 0.5% to 3% by weight of the total composition.

20. A topical gel composition according to any one of claims 9 to 19, wherein the moisturizer is selected from the group consisting of an emollient, an occlusive, a humectant and a mixture thereof.

21. A topical gel composition according to any one of claims 9 to 20, wherein the moisturizer is selected from the group consisting of diisopropyl adipate, octyldodecanol (sold under the name Kollicream^{®} OD), Glycerides, Capric Triglyceride (sold under the name Kollisolv^{®} MCT 70), liquid Vaseline, propylene glycol and glycerine.

22. A topical gel composition according to any one of claims 9 to 21, wherein the moisturizer is optionally present in an amount from 20 % to 45 % by weight of the total composition, preferably from 30% to 40% by weight of the total composition.

23. A topical gel composition according to any one of claims 8 to 22, wherein the preservative is selected from the group consisting of phenoxyethanol, EDTA, Salinip XB, methylparaben sodium, propylparaben sodium, benzyl alcohol, methylparaben and propylparaben or a mixture thereof.

24. A topical gel composition according to any one of claims 8 to 23, wherein the preservative is present in an amount from 0.5 % to 1.5 % by weight of the total composition.
